# EUROPEAN PATENT APPLICATION

(11) **EP 3 632 465 A1**
(43) Date of publication of application: **08.04.2020**
(21) Application number: 18198484.0
(22) Date of filing: 03.10.2018
(51) Int. Cl.: A61K 39/05, A61K 39/08, A61K 39/095, A61K 39/02, A61K 39/12, A61P 31/04

(54) **COMBINED IMMUNIZATION AGAINST MENINGOCOCCAL DISEASE AND HUMAN PAPILLOMAVIRUS**

(71) Applicant: Sanofi Pasteur Inc., Swiftwater, PA 18370 (US)
(72) Inventor: The designation of the inventor has not yet been filed
(74) Representative: Sanofi

(57) **Abstract**

Provided herein are compounds, compositions, formulations, kits, uses, and methods for immunization against *Neisseria meningitidis* serogroups A, C, Y, and W-135 and human papilloma virus.

## Description

### I. INTRODUCTION AND SUMMARY

*Neisseria meningitidis* (*N. meningitidis*) is a leading cause of bacterial meningitis and sepsis throughout the world. Serogroups A, C, Y, and W-135 of *Neisseria meningitidis* (MenA, MenC, MenY, and MenW, respectively; collectively referred to as MenACYW) are responsible for a substantial portion of meningococcal diseases worldwide. There are currently six types of vaccines to protect against *N. meningitidis* - quadrivalent meningococcal conjugate vaccines such as Menactra®, Nimenrix®, and Menveo®; meningococcal polysaccharide vaccine such as Menomune®, Serogroup C meningococcal vaccines such as Neisvac-C®, Menjugate® and Menitorix®, Serogroup A meningococcal vaccines such as MenAfriVac®, Serogroups C and Y meningococcal vaccines such as MenHibrix®, and Serogroup B meningococcal vaccines such as Bexsero® and Trumenba®. The epidemiology of *N. meningitidis* can be described as complex, unpredictable, geographically variable and changing over time.

Meningococcal vaccines may be conjugated to carrier proteins, such as diptheria toxin (DT), tetanus toxoid (TT), or CRM197, a non-toxic mutant of diptheria toxin, which may alter the host immune response to the administered vaccine. For example, Menactra® is formulated with DT, Menveo® with CRM197, and Nimenrix® with TT.

Human papillomavirus (HPV) is a group of viruses associated with various cancers most notably including cervical cancer, as well as warts, precancerous lesions, and laryngeal papillomatosis. HPV16 and HPV18 are particularly associated with cervical cancer, while HPV6 and HPV11 are associated with warts and laryngeal papillomatosis. Gardasil™ (quadrivalent human papillomavirus [type 6, 11, 16, 18] (HPV) recombinant vaccine) (Merck & Co Inc) is an HPV vaccine comprising an HPV 6 L1 protein, an HPV11 L1 protein, an HPV 16 L1 protein, and an HPV 18 L1 protein, along with an aluminum adjuvant. Gardasil™ can be administered as a 3-dose regimen according to a 0, 2, and 6-month schedule. Gardasil™ was originally approved in 2006 in the US and has been approved in at least 120 other countries. It has been recommended by the US FDA that Gardasil™ be administered before adolescence. *See* Markowitz et al., MMWR Recommendations and Reports 56 (RR-2): 1-24 (2007).

Vaccines are commonly administered in combination (e.g., during the same visit to a medical professional) in the US, Europe, and many other countries, e.g., to minimize the number of visits needed to fully immunize an individual against all desired pathogens. As such, it is desirable to use combinations of vaccines that do not significantly interfere with each other when coadministered. Whether two vaccines will be mutually non-interfering can be difficult to predict in advance and generally must be evaluated through clinical trials. Additionally, coadministering multiple substances can also increase the risk of side effects such as swelling, pain, and other undesired phenomena.

Several clinical trials have been conducted to evaluate coadministration of various combinations of *N. meningitidis* and HPV vaccines and reported either or both of data indicative of interference or an increased risk of at least one undesired side effect such as swelling or pain.

For example, in the clincal trial designated NCT00518180 available through ClinicalTrials.gov, Menveo™, Gardasil™, and Tdap were coadministered or administered separately. Geometric mean titers (GMT) for all four of HPV Types 6, 11, 16, and 18 in the coadministration group were below the lower bound of the 95% confidence interval for the population that received HPV alone. Similarly, in the trial described in European Medicines Agency (EMEA) Assessment report EMA-CHMP-233457-2018 (22-Mar-2018), Menveo™ and Gardasil™ were coadministered or administered separately. The GMTs against HPV-16 and HPV-18 for the coadministration group were both below the lower bound of the confidence interval for GMTs against HPV-16 and HPV-18 in the HPV only group, suggesting interference.

In another example, in the trial described in EMEA Assessment report EMA/100422/2018 (25-Jan-2018), Nimenrix™ and Cervarix™ (comprising proteins from two HPV types, 16 and 18) were coadministered. Anti-HPV-18 GMT values were 4306 for the coadministration group and 5655 for the HPV/nonconcomitant group, showing a decrease of about 24% upon coadministration. Although the assessment report does not provide confidence intervals, it is likely that this decrease indicates that interference occurs and, in any event, does not support a conclusion of noninterference.

In still another example, in the trial described in Schilling et al., Pediatrics (2015) 136, e563, Menactra™ and Gardasil-9™ (comprising proteins from the four HPV types in Gardasil™ and five additional types) were coadministered. The coadministration group experienced significantly more swelling than the nonconcomitant group.

Accordingly, a need exists for uses and methods for concurrent immunization against *N. meningitidis* and HPV. In particular, existing uses and methods may result in a weakened seroresponse to one or more of an HPV, such as HPV type 6, 11, 16, or 18, or *N. meningitidis,* such as *N. meningitidis* serotype A, C, Y, or W-135.

In some embodiments, compositions for use, methods, and/or uses disclosed herein provide one or more benefits, or at least provide the public with a useful choice. Such benefits can include immunogenicity against both *N. meningitidis,* such as N. *meningitidis* serotype A, C, Y, and/or W-135, and HPV, such as HPV type 6, 11, 16, and/or 18, wherein the administration of *N. meningitidis* antigens does not interfere with the development of immunity against HPV and/or the administration of HPV antigens does not interfere with the development of immunity against *N. meningitidis,* and/or wherein coadministration does not increase the risk of swelling or another side effect relative to a separate administration, e.g., of the HPV protein or proteins from types 6, 11, 16, and/or 18.

Accordingly, the following embodiments are provided. Embodiment 1 is a *Neisseria meningitidis* vaccine composition for use in a method of immunization against *Neisseria meningitidis* serogroups A, C, Y, and W-135 and human papilloma virus, wherein: the *Neisseria meningitidis* vaccine composition comprises:
a) a first conjugate of MenA capsular polysaccharide to tetanus toxoid;
b) a second conjugate of MenC capsular polysaccharide to tetanus toxoid;
c) a third conjugate of MenW-135 capsular polysaccharide to tetanus toxoid; and
d) a fourth conjugate of MenY capsular polysaccharide to tetanus toxoid;
the method comprises coadministering the *Neisseria meningitidis* vaccine composition and an HPV Type 18 L1 protein to a subject in need thereof; and
the administration of the *Neisseria meningitidis* vaccine composition does not interfere with the development of immunity against HPV Type 18.

Embodiment 2 is the *Neisseria meningitidis* vaccine composition for use according to embodiment 1, wherein the method further comprises coadministering an HPV Type 16 L1 protein with the *Neisseria meningitidis* vaccine composition.

Embodiment 3 is the *Neisseria meningitidis* vaccine composition for use according to any one of the preceding embodiments, wherein the administration of the *Neisseria meningitidis* vaccine composition does not interfere with the development of immunity against HPV Type 16.

Embodiment 4 is the *Neisseria meningitidis* vaccine composition for use according to any one of the preceding embodiments, wherein the method further comprises coadministering an HPV Type 6 L1 protein and/or an HPV Type 11 L1 protein with the *Neisseria meningitidis* vaccine composition.

Embodiment 5 is the *Neisseria meningitidis* vaccine composition for use according to any one of the preceding embodiments, wherein the administration of the *Neisseria meningitidis* vaccine composition does not interfere with the development of immunity against HPV Type 6 and/or HPV Type 11.

Embodiment 6 is the *Neisseria meningitidis* vaccine composition for use according to any one of the preceding embodiments, wherein the administration of the HPV protein or proteins does not interfere with the development of immunity against *Neisseria meningitidis* serogroups A, C, Y, and/or W-135.

Embodiment 7 is the *Neisseria meningitidis* vaccine composition for use according to any one of the preceding embodiments, wherein interference or non-interference with the development of immunity against HPV Type 18, 16, 11, and/or 6 and/or *Neisseria meningitidis* serogroups A, C, Y, and/or W-135 is determined by comparing geometric mean titers.

Embodiment 8 is the *Neisseria meningitidis* vaccine composition for use according to any one of the preceding embodiments, wherein the coadministration of the *Neisseria meningitidis* vaccine composition and the HPV protein or proteins does not result in increased risk of injection site swelling relative to administration of the HPV protein or proteins without the *Neisseria meningitidis* vaccine composition.

Embodiment 9 is the *Neisseria meningitidis* vaccine composition for use according to any one of the preceding embodiments, wherein the method further comprises coadministering a diphtheria-tetanus-pertussis vaccine with the *Neisseria meningitidis* vaccine composition, optionally wherein the diphtheria-tetanus-pertussis vaccine is Tetanus, diphtheria, acellular pertussis [Tdap] vaccine or DTaP5.

Embodiment 10 is the *Neisseria meningitidis* vaccine composition for use according to embodiment 9, wherein the administration of the diphtheria-tetanus-pertussis vaccine does not interfere with the development of immunity against *Neisseria meningitidis* serogroups A, C, Y, and/or W-135.

Embodiment 11 is the *Neisseria meningitidis* vaccine composition for use according to any one of the preceding embodiments, wherein the subject is a male or female aged from 8 to 25 years, 8 to 21 years, 8 to 18 years, 9 to 17 years, 9 to 25 years, 9 to 21 years, 9 to 19 years, 9 to 17 years, 10 to 25 years, 10 to 21 years, 10 to 18 years, 10 to 17 years, 8 to 14 years, 9 to 14 years, or 10 to 14 years.

Embodiment 12 is the *Neisseria meningitidis* vaccine composition for use according to any one of the preceding embodiments, wherein the coadministration of the *Neisseria meningitidis* vaccine composition is with the first dose of the HPV protein or proteins, and/or wherein the HPV protein or proteins are administered as part of a series of three doses.

Embodiment 13 is the *Neisseria meningitidis* vaccine composition for use according to any one of the preceding embodiments, wherein the *Neisseria meningitidis* vaccine composition and the HPV protein or proteins are administered intramuscularly and/or to different sites.

Embodiment 14 is the *Neisseria meningitidis* vaccine composition for use according to any one of the preceding embodiments, wherein:
(i) the second conjugate is a population comprising double-end-linked conjugated polysaccharides and single-end-linked conjugated polysaccharides which both are attached to the tetanus toxoid through a secondary amine, and/or the polysaccharides of the second conjugate have an O-acetylation level of 0.3 µmol/mg polysaccharide to 1.6 µmol/mg polysaccharide;
(ii) the second conjugate is a population comprising single-end-linked conjugated polysaccharides which are attached to the tetanus toxoid through a secondary amine, wherein the single-end-linked conjugated polysaccharides have a terminal unlinked saccharide, optionally wherein the terminal saccharide has a primary hydroxyl or secondary amine linkage at the 7 position, or wherein the reducing end is modified with a (2-hydroxy)ethoxy or secondary amine linkage;
(iii) the MenA capsular polysaccharide is attached to the tetanus toxoid through a linker comprising a carbamate, a spacer, and an amide, wherein the spacer is between the carbamate and the amide and comprises 2-10 linear carbons, and/or the first conjugate has a polysaccharide to tetanus toxoid mass ratio of 0.3 to 1.5;
(iv) the MenA capsular polysaccharide is attached to the tetanus toxoid through a linker comprising a carbamate, a spacer, and an amide, optionally wherein the spacer is between the carbamate and the amide and comprises 2-10 linear carbons;
(v) the MenC, MenW-135, and MenY capsular polysaccharides are attached to the tetanus toxoid through a secondary amine; and/or at least one of the conjugates has a weight average molecular weight ranging from 300 kDa to 1500 kDa;
(vi) one or more of the first, second, third, and fourth conjugates has a weight average molecular weight ranging from 300 kDa to 1500 kDa; and/or the composition comprises less than 20% free polysaccharide by weight relative to total polysaccharide; and/or
(vii) one or more of the first, second, third, and fourth conjugates have a polysaccharide to tetanus toxoid mass ratio of 0.3 to 1.5; and/or the composition comprises less than 20% free polysaccharide by weight relative to total polysaccharide.

Embodiment 15 is the *Neisseria meningitidis* vaccine composition for use according to any one of the preceding embodiments, wherein the first, second, third, and/or fourth conjugates are a population comprising molecules with a molecular weight in the range of 700 kDa to 1400 kDa or 800 kDa to 1300 kDa.

Embodiment 16 is the *Neisseria meningitidis* vaccine composition for use according to any one of embodiments 14 or 15, wherein molecular weight is determined by multi-angle light scattering (MALS).

Embodiment 17 is the *Neisseria meningitidis* vaccine composition for use according to any one of the preceding embodiments, wherein the MenC polysaccharide has a degree of O-acetylation ranging from 0.6 to 1.5 µmol/mg polysaccharide or 0.8 to 1.4 µmol/mg polysaccharide.

Embodiment 18 is the *Neisseria meningitidis* vaccine composition for use according to any one of the preceding embodiments, wherein the conjugate comprising MenC polysaccharide is a population comprising double-end-linked conjugated polysaccharides and single-end-linked conjugated polysaccharides, optionally wherein the single-end-linked polysaccharides of the second conjugate comprise a terminal unlinked saccharide, wherein the single-end-linked conjugated polysaccharides have a terminal unlinked saccharide, wherein the terminal saccharide has a primary hydroxyl at the 7 position, or wherein the reducing end is modified with a (2-hydroxy)ethoxy.

Embodiment 19 is the *Neisseria meningitidis* vaccine composition for use according to any one of the preceding embodiments, wherein the conjugate comprising MenC polysaccharide comprises one or more modifications chosen from (i) a primary hydroxyl at the 7 position, (ii) a (2-hydroxy)ethoxy at the reducing end, and (iii) a conjugation to the tetanus toxoid, wherein the modifications are present at no less than 25 nmol/mg polysaccharide; and/or the conjugate of MenW-135 and/or MenY polysaccharide comprises one or more modifications chosen from (i) a primary hydroxyl at a position of a vicinal diol in a native MenW-135 or MenY polysaccharide and (ii) a conjugation to the tetanus toxoid, wherein the modifications are present at no less than 60 nmol/mg polysaccharide.

Embodiment 20 is the *Neisseria meningitidis* vaccine composition for use according to any one of the preceding embodiments, wherein the MenC polysaccharide is reduced in size by 3x-8x relative to native MenC polysaccharide.

Embodiment 21 is the *Neisseria meningitidis* vaccine composition for use according to any one of the preceding embodiments, wherein:
(i) the conjugate of the MenA capsular polysaccharide to the tetanus toxoid has a polysaccharide to tetanus toxoid mass ratio of 0.5 to 1.5, 0.7 to 1.4, or 0.8 to 1.3;
(ii) the conjugate of the MenC capsular polysaccharide to the tetanus toxoid has a polysaccharide to tetanus toxoid mass ratio of 0.3 to 1.1 or 0.4 to 0.8;
(iii) the conjugate of the MenY capsular polysaccharide to the tetanus toxoid has a polysaccharide to tetanus toxoid mass ratio of 0.3 to 1.1, 0.5 to 1.3, or 0.5 to 0.9; and/or
(iv) the conjugate of MenW-135 capsular polysaccharide to the tetanus toxoid has a polysaccharide to tetanus toxoid mass ratio of 0.3 to 1.3 or 0.6 to 1.3.

Embodiment 22 is the *Neisseria meningitidis* vaccine composition for use according to any one of the preceding embodiments, wherein the composition comprises less than 20% free polysaccharide by weight, less than 10% free polysaccharide by weight, less than 5% free polysaccharide by weight, or substantially lacks free polysaccharide.

Embodiment 23 is the *Neisseria meningitidis* vaccine composition for use according to any one of the preceding embodiments, wherein the polysaccharide of the MenA, MenC, MenW-135, or MenY conjugate is attached to the tetanus toxoid through a linker.

Embodiment 24 is the *Neisseria meningitidis* vaccine composition for use according to embodiment 23, wherein:
(i) the linker comprises 2-10 linear carbons;
(ii) the linker is present in the MenA, MenC, MenW-135, or MenY conjugate at a ratio of one linker per 10-100 saccharide repeat units or 20-60 saccharide repeat units; and/or
(iii) the linker comprises a spacer between a first carbonyl and a second carbonyl, and the spacer comprises 4-8 carbons.

Embodiment 25 is the *Neisseria meningitidis* vaccine composition for use according to any one of the preceding embodiments, wherein the MenA conjugate comprises a linker comprising a residue of a dihydrazide or a residue of adipic acid dihydrazide.

Embodiment 26 is the *Neisseria meningitidis* vaccine composition for use according to any one of the preceding embodiments, wherein the polysaccharide of the MenA conjugate is attached to the tetanus toxoid through a linker of formula I: 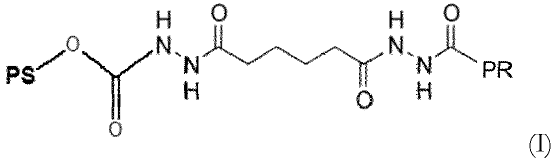 wherein PS indicates attachment to the polysaccharide and PR indicates attachment to the tetanus toxoid.

Embodiment 27 is the *Neisseria meningitidis* vaccine composition for use according to any one of the preceding embodiments, wherein the polysaccharide of the MenC, MenW-135, and/or MenY conjugate is attached to the tetanus toxoid as shown in formula II:
PR-NH-CH₂-PS (II)
wherein PS indicates attachment to the polysaccharide and PR indicates attachment to the tetanus toxoid.

Embodiment 28 is the *Neisseria meningitidis* vaccine composition for use according to any one of the preceding embodiments, wherein the *Neisseria meningitidis* vaccine composition is a single unit dose composition comprising from 6 µg to 15 µg or 4 µg to 10 µg of each of the MenA, MenC, MenW-135, and MenY polysaccharides, and/or wherein the tetanus toxoid is present in the vaccine composition in an amount from 50 µg to 80 µg.

Embodiment 29 is the *Neisseria meningitidis* vaccine composition for use according to any one of the preceding embodiments, wherein the subject did not previously receive a *Neisseria meningitidis* capsular saccharide conjugate vaccine and/or did not previously receive an HPV vaccine.

Embodiment 30 is the *Neisseria meningitidis* vaccine composition for use according to any one of the preceding embodiments, wherein the HPV Type 18 L1 protein is administered as part of an HPV vaccine.

Embodiment 31 is the *Neisseria meningitidis* vaccine composition for use according to embodiment 30, wherein the HPV vaccine comprises one, two, three, or all of:
(i) a 20 µg dose of HPV Type 6 L1 protein;
(ii) a 40 µg dose of HPV Type 11 L1 protein;
(iii) a 40 µg dose of HPV Type 16 L1 protein; and/or
(iv) a 20 µg dose of HPV Type 18 L1 protein.

Embodiment 32 is the *Neisseria meningitidis* vaccine composition for use according to embodiment 30 or 31, wherein the HPV vaccine comprises one, two, three, four, or all of:
(i) amorphous aluminum hydroxyphosphate sulfate adjuvant;
(ii) sodium chloride;
(iii) L-histidine;
(iv) polysorbate 80; and/or
(v) sodium borate.

Embodiment 33 is the *Neisseria meningitidis* vaccine composition for use according to any one of embodiments 30-32, wherein the HPV vaccine is quadrivalent human papillomavirus [type 6, 11, 16, 18] (HPV) recombinant vaccine (Gardasil™).

Embodiment 34 is the *Neisseria meningitidis* vaccine composition for use according to any one of the preceding embodiments, wherein the HPV L1 protein or proteins are in the form of virus-like particles.

Additional embodiments are provided below.

### II. BRIEF DESCRIPTION OF THE FIGURES

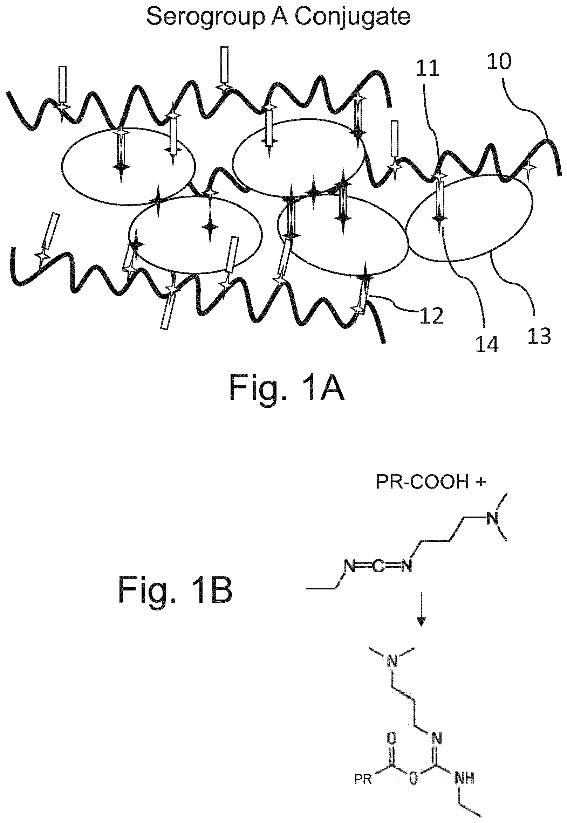

**Figure 1A** shows a schematic of a Serogroup A Polysaccharide-ADH linked protein Conjugate. Serogroup A polysaccharides **10** with reactive site residues **11** formed by activation with carbonyl diimidazole (CDI) and optionally derivatization with adipic acid dihydrazide (ADH) at hydroxyl groups of the polysaccharide and reaction with a protein (e.g., Tetanus Toxoid (TT)). Activated/derivatized polysaccharide is crosslinked to the protein **13** through a linkage **12** directly or indirectly at groups **14.** For example, direct linkages can use primary amines of the protein, e.g., by forming a carbamate linkage (e.g., derived from CDI). Indirect linkages can be derived from ADH and *N*-Ethyl-*N*-(3-dimethylaminopropyl)carbodiimide (EDAC), which activates carboxyls of the protein.
**Figure 1B** illustrates preparation of an active O-acylisourea intermediate of a carrier protein (e.g., TT) using *N*-Ethyl-*N'*-(3-dimethylaminopropyl)carbodiimide (EDAC), which reacts with carboxyl groups (e.g., on aspartic acid or glutamic acid side chains, or the C-terminus) of the protein. This intermediate is suitable for coupling to amine groups of an activated derivatized polysaccharide (not shown).

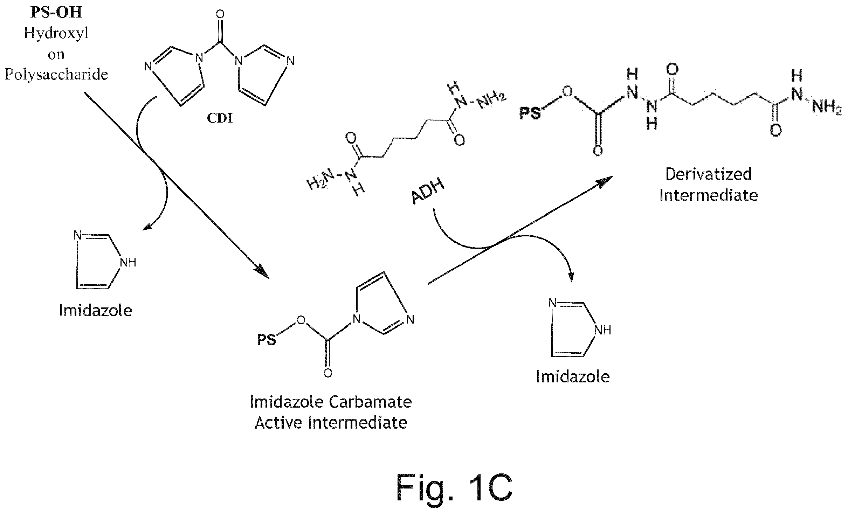

**Figure 1C** illustrates a general scheme for producing activated derivatized Serogroup A polysaccharide, which can be used to produce a Serogroup A Polysaccharide-ADH Conjugate linked to a carrier protein (e.g., TT). In this embodiment, polysaccharides are activated at hydroxyl groups with CDI, forming an imidazole carbamate active intermediate, which is further derivatized with ADH. The ADH-derivatized Serogroup A polysaccharide is suitable for covalent attachment to the carrier protein via amine coupling of the primary amine groups on the ADH linker to an active O-acylisourea intermediate of the carrier protein (not shown).

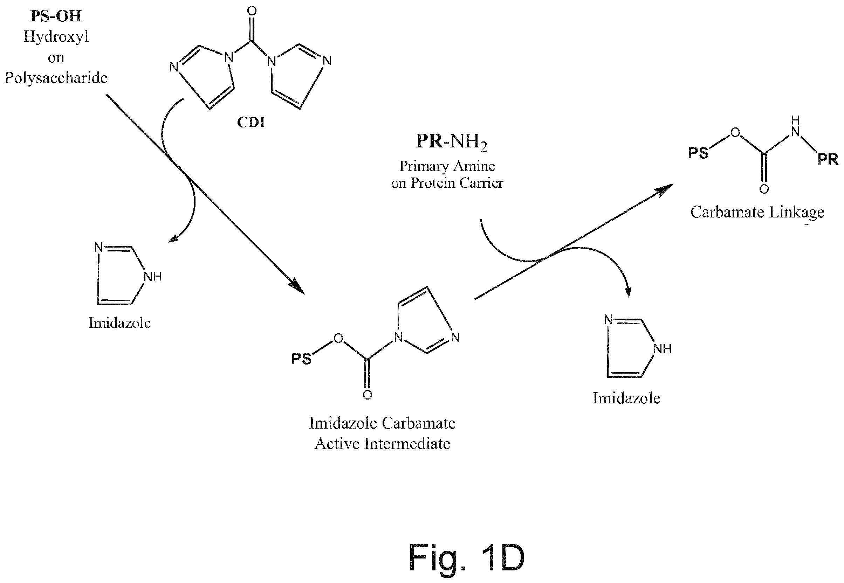

**Figure 1D** shows a general scheme for producing a Serogroup A Polysaccharide linked to Tetanus Toxoid Conjugate via a carbamate. Polysaccharides (PS) are activated at hydroxyl groups with CDI, forming an imidazole carbamate active intermediate. The active intermediate is then reacted with a protein carrier (PR). A carbamate linkage is formed through a nucleophilic substitution reaction in which a primary amine of the protein attacks the carbamate carbon, resulting in loss of imidazole and formation of a carbamate linkage between the polysaccharide and protein.

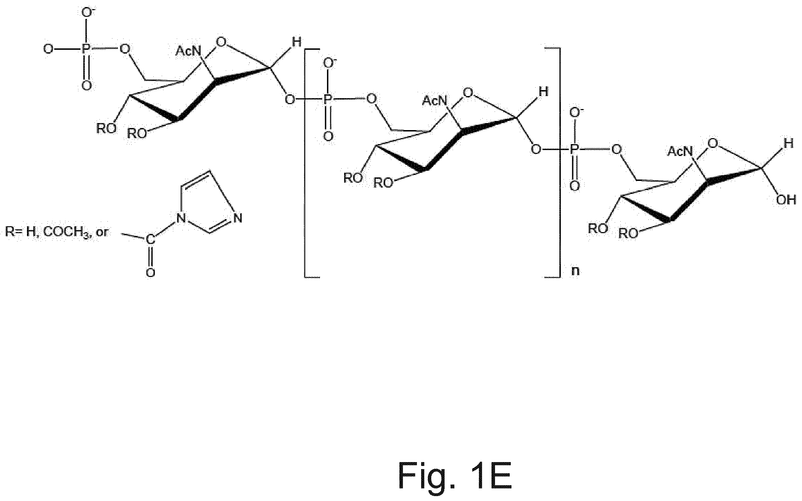

**Figures 1E-F** show the structure of a Serogroup A polysaccharide (E) following CDI-activation and (F) following CDI-activation and derivatization with ADH.

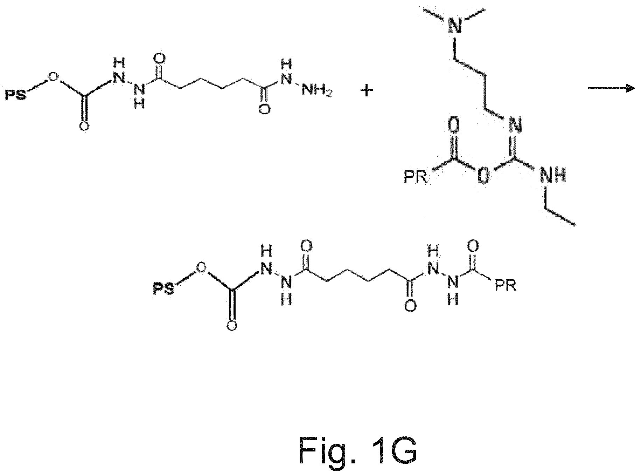

**Figure 1G** illustrates producing a Serogroup A Polysaccharide-ADH Conjugate linked to a carrier protein (e.g., TT) from activated derivatized polysaccharide and an active O-acylisourea intermediate formed from Tetanus Toxoid carrier protein and *N*-Ethyl-*N'*-(3-dimethylaminopropyl)carbodiimide (EDAC). The primary amine of the activated derivatized polysaccharide substitutes for the isourea, which serves as a leaving group, giving a product in which the protein is linked to the polysaccharide through an amide bond, the residue of ADH, and a carbamate linkage, in which the carbonyl is derived from CDI. The eliminated urea by-product is not shown.

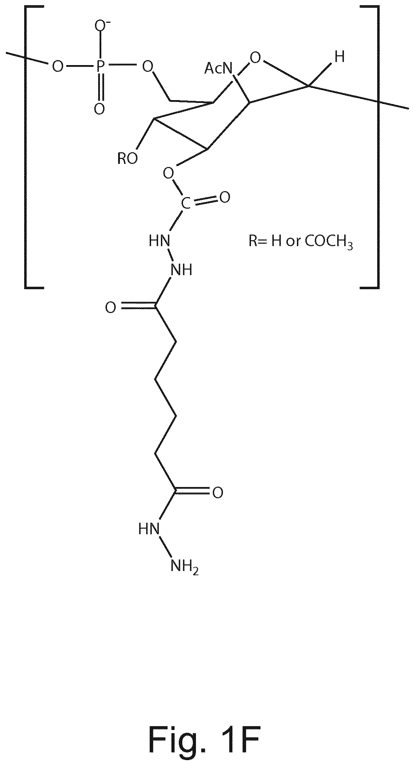

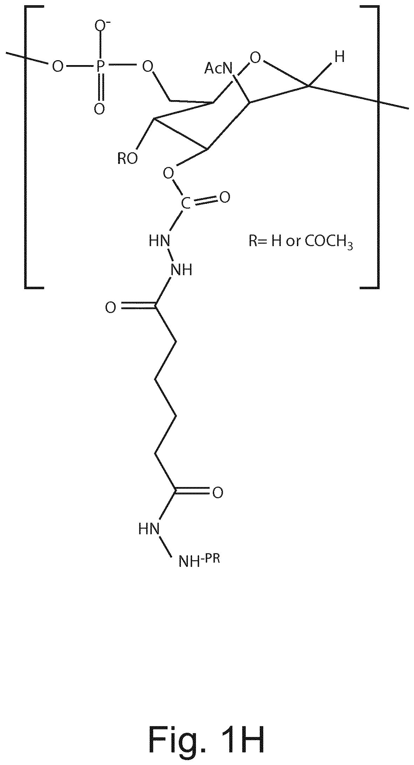

**Figure 1H** shows the product of the reaction in FIG. 1F with the structure of the linked polysaccharide repeat unit (including ADH residue) drawn out.

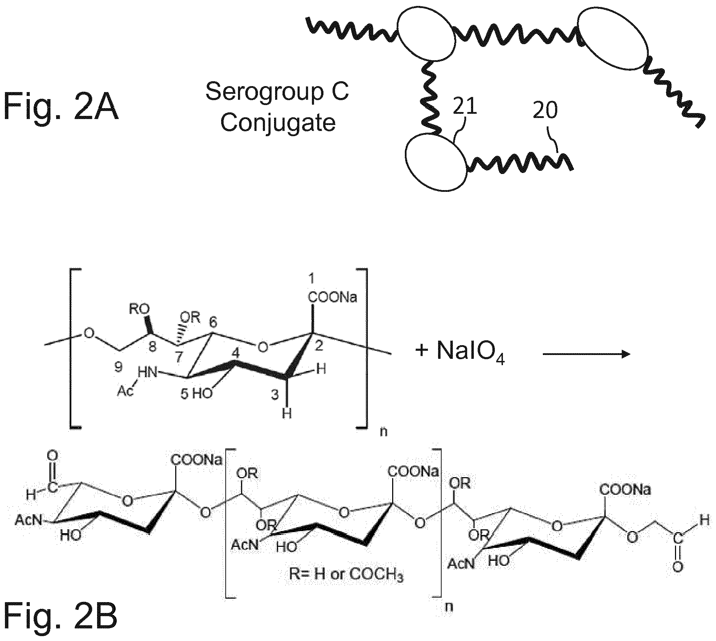

**Figure 2A** shows a schematic of a Serogroup C Polysaccharide-Protein Conjugate. Serogroup C polysaccharides **20** are bonded to a protein (e.g., Tetanus Toxoid) **21** at their termini.
**Figure 2B** illustrates activation of Serogroup C Polysaccharide (shown with conventional numbering of the carbons in the polysaccharide repeat unit) using sodium metaperiodate. Sodium metaperiodate treatment results in cleavage between the 7 and 8 carbons, oxidatively depolymerizing the polysaccharide into corresponding terminal aldehydes.

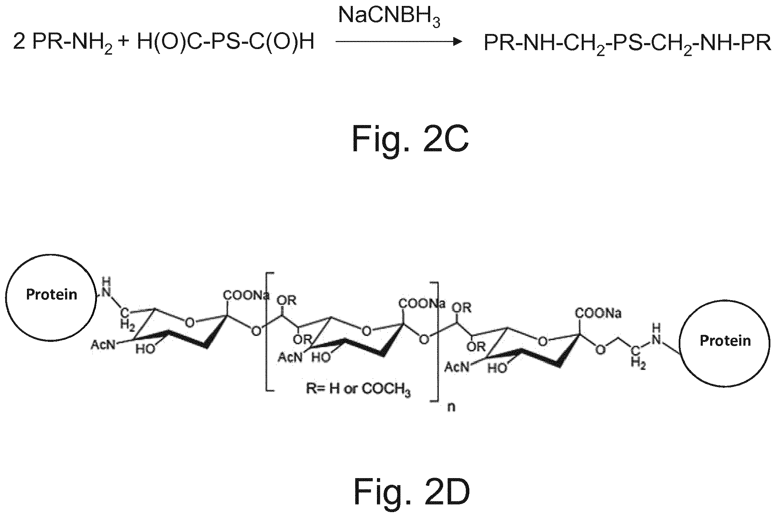

**Figure 2C** illustrates formation of a Serogroup C Polysaccharide-protein (e.g., TT) conjugate via reductive amination. A primary amine of the protein (PR) (e.g., lysine side chain or N-terminus) reacts with a terminal aldehyde of a depolymerized, activated Serogroup C Polysaccharide (PS) to form a Schiff base intermediate (not shown), which is reduced (e.g., using pyridine borane, picoline borane, or a cyanoborohydride) to give a secondary amine linkage. The polysaccharide moiety is end-linked to the protein. Individual protein molecules may react with more than one polysaccharide and some polysaccharide termini may be unreacted (not shown; see illustration in FIG. 2A). Unreacted aldehydes can be capped, i.e., reduced to alcohols, using a suitable reducing agent such as sodium borohydride after reduction of the Schiff base (not shown).
**Figure 2D** shows the product of the reaction in FIG. 2C with the structure of the linked polysaccharide repeat unit drawn out. Linkage of the protein to additional polysaccharides is possible (not shown).

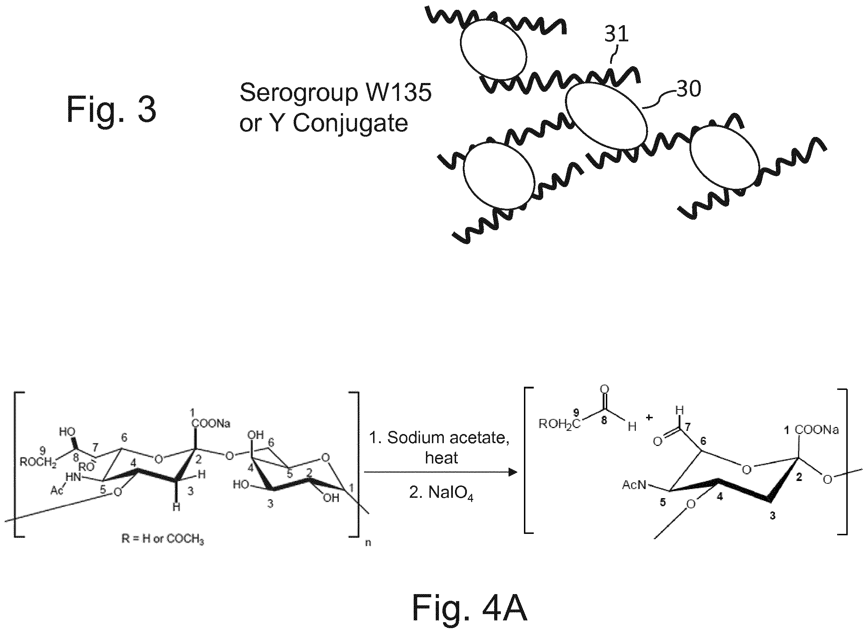

**Figure 3** shows a schematic of a Serogroup W-135 or Serogroup Y Polysaccharide-Protein Conjugate. Serogroup W-135 or Serogroup Y polysaccharides **31** are bonded to one or more proteins (e.g., Tetanus Toxoid) **30** at one or more positions.
**Figure 4A** illustrates depolymerization and activation of Serogroup W-135 Polysaccharide. The polysaccharide is depolymerized using, e.g., elevated temperature and then activated by treatment with sodium metaperiodate, which cleaves vicinal diols such as, for example, between carbon 7 and 8 of the sialic acid moiety and oxidizes them to aldehydes.

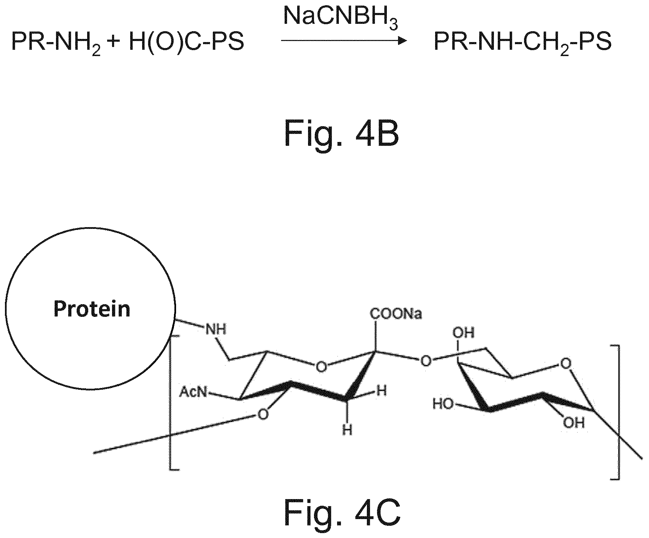

**Figure 4B** illustrates formation of a Serogroup W-135 Polysaccharide-protein (e.g., TT) conjugate via reductive amination. A primary amine of the protein (PR) (e.g., lysine side chain or N-terminus) reacts with an aldehyde of a depolymerized, activated Serogroup W-135 Polysaccharide (PS) to form a Schiff base intermediate (not shown). The intermediate is reduced by sodium cyanoborohydride to give a secondary amine linkage. Individual protein molecules may react with more than one polysaccharide and vice versa (not shown; see illustration in FIG. 3). Unreacted aldehydes can be capped, i.e., reduced to alcohols, using a suitable reducing agent such as sodium borohydride after reduction of the Schiff base (not shown).
**Figure 4C** shows a product of the reaction in FIG. 4B with one possible structure of the linked polysaccharide repeat unit drawn out. Linkage of the protein to additional polysaccharides or vice versa are possible (not shown; see illustration in FIG. 3).

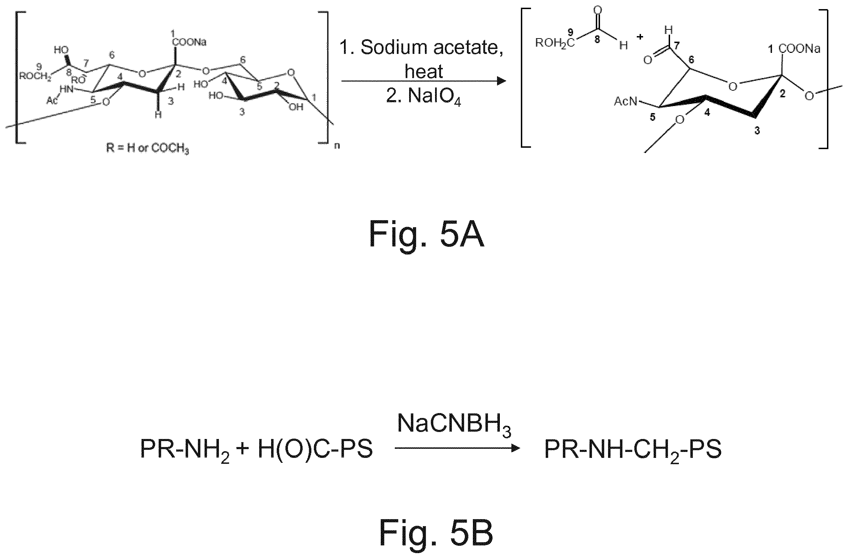

**Figure 5A** illustrates depolymerization and activation of Serogroup Y Polysaccharide. The polysaccharide is depolymerized using, e.g., elevated temperature and then activated by treatment with sodium metaperiodate, which cleaves vicinal diols such as, for example, between carbon 7 and 8 of the sialic acid moiety and oxidizes them to aldehydes.
**Figure 5B** illustrates formation of a Serogroup Y Polysaccharide-protein (e.g., TT) conjugate via reductive amination. A primary amine of the protein (PR) (e.g., lysine side chain or N-terminus) reacts with an aldehyde of a depolymerized, activated Serogroup Y Polysaccharide (PS) to form a Schiff base intermediate (not shown). The intermediate is reduced (e.g., using pyridine borane, picoline borane, or a cyanoborohydride) to give a secondary amine linkage. Individual protein molecules may react with more than one polysaccharide and vice versa (not shown; see illustration in FIG. 3). Unreacted aldehydes can be capped, i.e., reduced to alcohols, using a suitable reducing agent such as sodium borohydride after reduction of the Schiff base (not shown).

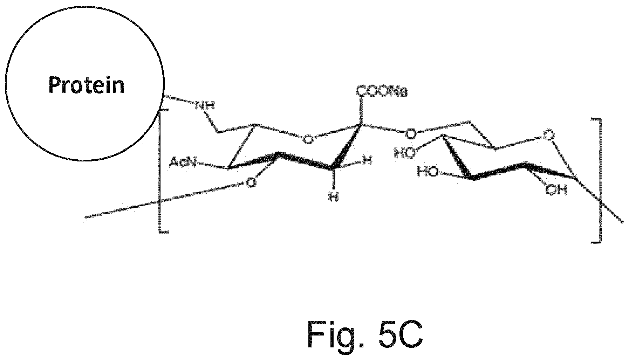

**Figure 5C** shows a product of the reaction in FIG. 5B with one possible structure of the linked polysaccharide repeat unit drawn out. Linkage of the protein to additional polysaccharides or vice versa are possible (not shown; see illustration in FIG. 3).

### III. DETAILED DESCRIPTION OF EMBODIMENTS OF THE INVENTION

Reference will now be made in detail to certain embodiments of the invention, examples of which are illustrated in the accompanying drawings. While the invention will be described in conjunction with the illustrated embodiments, it will be understood that they are not intended to limit the invention to those embodiments. On the contrary, the invention is intended to cover all alternatives, modifications, and equivalents, which may be included within the invention as defined by the appended claims.

Before describing the present teachings in detail, it is to be understood that the disclosure is not limited to specific compositions or process steps, as such may vary. It should be noted that, as used in this specification and the appended claims, the singular form "a", "an" and "the" include plural references unless the context clearly dictates otherwise. Thus, for example, reference to "a conjugate" includes a plurality of conjugates and reference to "a cell" includes a plurality of cells and the like.

Numeric ranges are inclusive of the numbers defining the range. Measured and measureable values are understood to be approximate, taking into account significant digits and the error associated with the measurement. Also, the use of "comprise", "comprises", "comprising", "contain", "contains", "containing", "include", "includes", and "including" are not intended to be limiting. It is to be understood that both the foregoing general description and detailed description are exemplary and explanatory only and are not restrictive of the teachings.

Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims).

The section headings used herein are for organizational purposes only and are not to be construed as limiting the desired subject matter in any way. In the event that any literature incorporated by reference contradicts any term defined in this specification, this specification controls. While the present teachings are described in conjunction with various embodiments, it is not intended that the present teachings be limited to such embodiments. On the contrary, the present teachings encompass various alternatives, modifications, and equivalents, as will be appreciated by those of skill in the art.

### A. Definitions

Unless stated otherwise, the following terms and phrases as used herein are intended to have the following meanings:

The term "or combinations thereof" as used herein refers to all permutations and combinations of the listed terms preceding the term. For example, "A, B, C, or combinations thereof" is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "kit" refers to a packaged set of related components, such as one or more compounds or compositions and one or more related materials such as solvents, solutions, buffers, instructions, or desiccants.

"Or" is used in the inclusive sense, i.e., equivalent to "and/or," unless the context requires otherwise.

The terms "linker" and "linkage" are used interchangeably and mean a chemical moiety comprising a chain of atoms that covalently attaches, or is attached to, items such as a carrier protein or a polysaccharide.

"Linking moiety" means a chemically reactive group, substituent or moiety, e.g. a nucleophile or electrophile, capable of reacting with another molecule to form a linkage by a covalent bond.

"Alkyl" means a saturated or unsaturated, branched, straight-chain, branched, or cyclic hydrocarbon radical derived by the removal of one hydrogen atom from a single carbon atom of a parent alkane, alkene, or alkyne. Typical alkyl groups consist of 1 to 12 saturated and/or unsaturated carbons, including, but not limited to, methyl, ethyl, propyl, butyl, and the like.

A "repeat unit" is the mono- or oligosaccharide residue that is polymerized in a polysaccharide. The repeat units of MenA and MenC are monosaccharides (N-acetyl mannosamine and sialic acid, respectively) and the repeat units of MenW-135 and MenY are disaccharides (of sialic acid and glucose for MenY, or sialic acid and galactose for MenW-135). Repeat units may vary from one to the next with respect to side chains (e.g., O-acetylation) and/or modifications such as those disclosed herein.

MenA, MenC, MenW-135, and MenY are used as shorthand for *Neisseria meningitidis* of serogroup A, C, W-135, or Y, respectively, or the capsular polysaccharide thereof (as in the case of, e.g., a "MenC conjugate" which means a conjugate of the capsular polysaccharide of *Neisseria meningitidis* of serogroup C to a carrier protein).

"Coadministration," "administered in combination," and similar terms and phrases mean that a first vaccine and a second vaccine are administered at approximately the same time, e.g., on the same day or during the same visit to a clinic or other medical service provider. The vaccines need not be administered to the same site (e.g., they could be administered in opposite shoulders) and may be, but are not necessarily, administered by the same route (e.g., intramuscular).

"Non-interference" and "does not interfere" mean, in the context of titers (e.g., geometric mean titers), that coadministration of a first vaccine with a second vaccine to a treatment group gives a titer specific for an antigen of the first vaccine where the lower bound of its 95% confidence interval is greater than two thirds of the titer of a control group given only the first vaccine. In the context of seroconversion, "non-interference" and "does not interfere" mean that coadministration of a first vaccine with a second vaccine to a treatment group gives an amount of seroconversion against an antigen of the first vaccine where the lower bound of its 95% confidence interval is greater than 90% of the seroconversion of a control group given only the first vaccine.

"Two-way non-interference" means both that a first vaccine does not interfere with a second vaccine and that a second vaccine does not interfere with a first vaccine.

"Does not result in increased risk" of a given side effect means that a given treatment (e.g., a coadministration) results in a frequency of the side effect less than or equal to the upper bound of the 95% confidence interval of the frequency of the side effect following a specified reference treatment (e.g., a separate administration of a component of what was coadministered).

### B. Methods and Vaccine Compositions for use in immunization

In some embodiments, a *Neisseria meningitidis* vaccine composition for use in a method of immunization against *Neisseria meningitidis* serogroups A, C, Y, and W-135 and human papilloma virus is provided, e.g., wherein the *Neisseria meningitidis* vaccine composition and an HPV L1 protein are coadministered. In some embodiments, the *Neisseria meningitidis* vaccine composition and an HPV vaccine comprising an HPV L1 protein are coadministered. As demonstrated in the Examples below, it has been found that *Neisseria meningitidis* vaccine compositions described herein can be coadministered with HPV L1 proteins, e.g., as in Gardasil™, without interference with the development of immunity against HPV and/or Neisseria meningitidis.

### 1. Exemplary Neisseria meningitidis vaccine compositions for uses disclosed herein

The *Neisseria meningitidis* vaccine composition for use as disclosed herein or used in a method disclosed herein can have any of the following features. In some embodiments, the *Neisseria meningitidis* vaccine composition comprises a conjugate of MenC capsular polysaccharide to a tetanus toxoid; a conjugate of MenA capsular polysaccharide to a tetanus toxoid; a conjugate of MenW-135 capsular polysaccharide to a tetanus toxoid; and a conjugate of MenY capsular polysaccharide to a tetanus toxoid.

Capsular polysaccharides may be prepared according to the method described in US 2003/0068336 Example 1. Capsular polysaccharides may also be prepared using the medium and methods described in US 6,933,137, for example.

General discussion of carrier proteins including tetanus toxoid may be found in, e.g., Pichichero ME. Protein carriers of conjugate vaccines: Characteristics, development, and clinical trials. Human Vaccines & Immunotherapeutics. 2013;9(12):2505-2523. dOi:10.4161 /hv.26109, which is incorporated herein by reference.

In some embodiments, the tetanus toxoid (TT) is prepared by extraction, ammonium sulfate purification, and formalin inactivation of the toxin from cultures of *Clostridium tetani* (Harvard Strain) grown in a Mueller and Miller medium or a modified Mueller and Miller medium. In some embodiments, the TT is processed to reduce residual formaldehyde, is concentrated in sodium chloride and is filter sterilized. In some embodiments, the TT is purified by chromatography rather than ammonium sulfate purification. In some embodiments, the modified Mueller and Miller medium does not contain beef heart infusion. In some embodiments, the *Clostridium tetani* is grown in the medium described in WO2006/042542 at Table 3, page 16.

Certain embodiments discussed below involve a feature, such as a chemical moiety (e.g., a hydroxyl or O-acetylation) or a conjugation to a carrier protein, which is present in a given amount per unit mass of polysaccharide. For example, a certain feature or combination of features may be present at a level such as no less than 25 nmol/mg polysaccharide. This means that in 1 mg of polysaccharide, the feature or combination of features occurs at least 15 x 10¹⁵ times (where 25 nmol = 25 x 10⁻⁹ mole x (6.02x10²³ items/mole) = 15 x 10¹⁵ items.

In some embodiments, the polysaccharides of the conjugate of MenC capsular polysaccharide to the carrier protein have an O-acetylation level ranging from 0.3 µmol/mg polysaccharide to 1.6 µmol/mg polysaccharide. In some embodiments, the level of O-acetylation is greater than or equal to 0.4, 0.5, 0.6, 0.7, 0.8, 0.9, 1.0, 1.1, or 1.2 µmol/mg polysaccharide. In some embodiments, the level of O-acetylation is less than or equal to 0.7, 0.8, 0.9, 1.0, 1.1, 1.2, 1.3, 1.4, or 1.5 µmol/mg polysaccharide. E.g., the level can range from 0.6 to 1.5 µmol/mg polysaccharide or 0.8 to 1.4 µmol/mg polysaccharide. O-acetyl content can be measured by the Hestrin method (Hestrin et. al., J. Biol. Chem. 1949, 180, p. 249).

In some embodiments, at least one of the conjugates in the composition has a weight-average molecular weight ranging from 300 kDa to 1500 kDa. In some such embodiments, the weight-average molecular weight is greater than or equal to 400 kDa, 500 kDa, 600 kDa, 700 kDa, 800 kDa, 900 kDa, 1000 kDa, or 1100 kDa. In some such embodiments, the weight-average molecular weight is less than or equal to 600 kDa, 700 kDa, 800 kDa, 900 kDa, 1000 kDa, 1100 kDa, 1200 kDa, 1300 kDa, or 1400 kDa. Weight-average molecular weight can be determined by methods known in the art, e.g., multi-angle light scattering (MALS). In some embodiments, at least one conjugate in the composition comprises (i.e., is a population of molecules comprising) molecules having a molecular weight in the range of 700 kDa to 1400 kDa. It should be noted that some molecules of the population can have a weight in the range regardless of whether the weight-average or number-average molecular weight is in the range or not. For example, a population of molecules with a weight-average molecular weight of 600 kDa or 1500 kDa will likely contain molecules having a molecular weight in the range of 700 kDa to 1400 kDa. In some embodiments, at least one conjugate in the composition comprises molecules having a molecular weight in the range of 800 kDa to 1300 kDa. In some embodiments, at least one conjugate in the composition comprises molecules having a molecular weight in the range of 700-800, 800-900, 900-1000, 1000-1100, 1100-1200, 1200-1300, 1300-1400, or 1400-1500 kDa. In some embodiments, a MenA conjugate has a molecular weight as described above. In some embodiments, a MenC conjugate has a molecular weight as described above. In some embodiments, a MenW-135 conjugate has a molecular weight as described above. In some embodiments, a MenY conjugate has a molecular weight as described above. In some embodiments, a molecular weight is determined using multi-angle light scattering (MALS). In some embodiments, a molecular weight is determined using high-performance size-exclusion chromatography (HPSEC).

In some embodiments, a conjugate, such as a conjugate of MenC, is a population comprising single-end-linked conjugated polysaccharides, double-end-linked polysaccharides, or a combination thereof. A single-end-linked conjugated polysaccharide is attached at one end to a carrier protein. A double-end-linked conjugated polysaccharide is attached at both ends to a carrier protein. End-linked conjugated polysaccharides can be formed, e.g., by cleaving and activating vicinal diols in the polysaccharide backbone to expose activated ends, such as by periodate treatment. For example, the MenC polysaccharide has a 7,8 vicinal diol in its sialic acid repeat unit to the extent that the 7 and 8 positions are not O-acetylated in the same repeat unit. This vicinal diol is in the backbone of the polysaccharide because cleaving it separates one saccharide from the next, i.e., the vicinal diol is not part of a side chain. Following activation, the activated ends can then react with a carrier protein (or to a linker which will then be or is already attached to a carrier protein), to form an end-linked polysaccharide conjugate. A conjugate is single-end linked if only one of the ends (terminal saccharide residues) of the polysaccharide is linked to a carrier protein (including through a linker if applicable). A double-end-linked conjugate has carrier proteins linked to both ends of the polysaccharide. In general, using a higher stoichiometry of polysaccharide relative to carrier protein or a lower overall reactant concentration will bias a conjugation reaction toward single-end-linked products. In contrast, a lower stoichiometry of polysaccharide relative to carrier protein or a higher overall reactant concentration will bias a conjugation reaction toward double-end-linked products.

In some embodiments, the single-end-linked conjugated polysaccharides (e.g., MenC conjugates) have a terminal unlinked saccharide in which there is a primary hydroxyl at the 7 position, or wherein the reducing end is modified with a (2-hydroxy)ethoxy. This can result from activation of a polysaccharide comprising 7,8 vicinal diols with periodate (which gives terminal aldehydes), conjugation at one end, and reduction of the unreacted aldehyde at the other end, e.g., with a borohydride reagent. The primary hydroxyl at the 7 position can be considered the end of a truncated sialic acid residue. The reducing end that is modified with a (2-hydroxy)ethoxy can be considered a sialic acid residue attached at its reducing end to a fragment of the 9 and 8 carbons of another residue and their associated oxygens.

In some embodiments, the conjugate is a MenW-135 and/or MenY polysaccharide that comprises one or more modifications chosen from (i) a primary hydroxyl at a position of a vicinal diol in a native MenW-135 or MenY polysaccharide and (ii) a conjugation to the carrier protein, wherein the modifications are present at no less than 60 nmol/mg polysaccharide. The modifications can be formed by periodate oxidation followed by conjugation to the carrier protein and reduction of unreacted aldehydes. Periodate-driven cleavage of the saccharide residues can occur at vicinal diol positions such as the 7,8 or 8,9 positions of the sialic acid and also potentially in the hexose ring of the repeat unit, particularly where the diols are in a cis arrangement. In some embodiments, the modifications are present in an amount less than 200 nmol/mg polysaccharide, less than 150 nmol/mg polysaccharide, less than 150 nmol/mg polysaccharide, less than 100 nmol/mg polysaccharide, or less than 80 nmol/mg polysaccharide.

In some embodiments, a polysaccharide is linked to a carrier protein through a secondary amine. In some embodiments, a polysaccharide is attached to the carrier protein as shown in formula II:
PR-NH-CH₂-PS (II)
wherein PS indicates attachment to the polysaccharide and PR indicates attachment to the carrier protein .Such a secondary amine linkage can be formed, for example, through reductive amination in which a primary amine on a protein (e.g., N-terminus or amino group of a lysine side chain) attacks an activated group (e.g., aldehyde) on a polysaccharide, forming a Schiff base which is then reduced to form the secondary amine. Reduction can be performed using a suitable reducing reagent such as a cyanoborohydride (e.g., sodium cyanoborohydride) or a borane (e.g., pyridine borane or picoline borane).

In some embodiments, a conjugate of a MenC polysaccharide is reduced in size by 3x-8x relative to native MenC polysaccharide, e.g., 3x-4x, 4x-5x, 5x-6x, 6x-7x, or 7x-8x. Periodate cleavage separates adjacent repeat units and thus provides for reduction in size of the polysaccharide. Size may be further reduced by a treatment such as heat and/or acid, e.g., before the periodate treatment. Other known treatments for reducing size may also be used, such as sonication or microfludization.

In some embodiments, a conjugate of a MenA polysaccharide has a polysaccharide to carrier protein mass ratio of 0.3 to 1.5, e.g., 0.3 to 0.4, 0.4 to 0.5, 0.5 to 0.6, 0.6 to 0.7, 0.7 to 0.8, 0.8 to 0.9, 0.9 to 1.0, 1.0 to 1.1, 1.1 to 1.2, 1.2 to 1.3, 1.3 to 1.4, or 1.4 to 1.5. In some embodiments, a conjugate of a MenA polysaccharide has a polysaccharide to carrier protein mass ratio of 0.5 to 1.5.

In some embodiments, a conjugate of a MenC polysaccharide has a polysaccharide to carrier protein mass ratio of 0.3 to 1.1, e.g., 0.3 to 0.4, 0.4 to 0.5, 0.5 to 0.6, 0.6 to 0.7, 0.7 to 0.8, 0.8 to 0.9, 0.9 to 1.0, or 1.0 to 1.1.

In some embodiments, a conjugate of a MenW-135 polysaccharide has a polysaccharide to carrier protein mass ratio of 0.3 to 1.3, e.g., 0.3 to 0.4, 0.4 to 0.5, 0.5 to 0.6, 0.6 to 0.7, 0.7 to 0.8, 0.8 to 0.9, 0.9 to 1.0, 1.0 to 1.1, 1.1 to 1.2, or 1.2 to 1.3.

In some embodiments, a conjugate of a MenY polysaccharide has a polysaccharide to carrier protein mass ratio of 0.5 to 1.3, e.g., 0.3 to 0.4, 0.4 to 0.5, 0.5 to 0.6, 0.6 to 0.7, 0.7 to 0.8, 0.8 to 0.9, 0.9 to 1.0, 1.0 to 1.1, 1.1 to 1.2, or 1.2 to 1.3.

In some embodiments, a vaccine composition provided herein comprises less than 20% free polysaccharide by weight, e.g., comprises less than 10% free polysaccharide by weight, less than 5% free polysaccharide by weight, or substantially lacks free polysaccharide. "Substantially lacks free polysaccharide" means that the level of free polysaccharide is below the detection limit of a deoxycholate precipitation assay in which protein-conjugated polysaccharide is precipitated with deoxycholate and polysaccharide remaining in solution is assayed, e.g., as described in Lei et al., "Quantification of free polysaccharide in meningococcal polysaccharide-diphtheria toxoid conjugate vaccines," Dev Biol (Basel). 2000; 103:259-64 (PMID: 11214246).

In some embodiments, a polysaccharide is attached to the carrier protein through a linker comprising 2-10 linear carbons, e.g., 2, 3, 4, 5, 6, 7, 8, 9, or 10 carbons. "Linear carbons" are carbons along the chain leading from the polysaccharide to the carrier protein and do not include carbons on a branch from this chain. In some embodiments, the linker comprises a spacer between a first carbonyl and a second carbonyl, and the spacer comprises 4-8 carbons (e.g., 4, 5, 6, 7, or 8 carbons), which may be linear carbons. The first carbonyl can be part of a carbamate. The second carbonyl can be part of an amide. The first carbonyl can be proximal to the polysaccharide and distal to the carrier protein. The second carbonyl can be proximal to the carrier protein and distal to the polysaccharide. The linker can comprise a residue of a dihydrazide, such as adipic acid dihydrazide (ADH). In some embodiments, the polysaccharide is attached to the carrier protein through a linker of formula I: 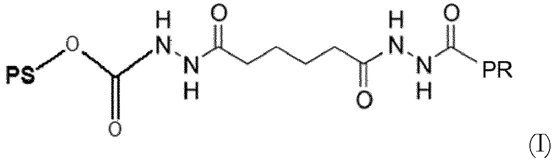 wherein PS indicates attachment to the polysaccharide and PR indicates attachment to the carrier protein. An individual polysaccharide can be attached to one or more than one carrier protein (at different positions), and vice versa.

In some embodiments, a linker is present in a conjugate at a ratio of one linker per 10-100 saccharide repeat units, e.g., 20-60. This includes linkers to which both a carrier protein and a polysaccharide are attached and also linkers attached only to the polysaccharide, i.e., which did not form an attachment to a carrier protein.

In some embodiments, a conjugate of a *Neisseria meningitidis* capsular polysaccharide to a carrier protein through a linker is provided in which the linker is present in an amount of 1 linker per 10-100 repeat units of the polysaccharide, e.g., 1 linker per 10-20 repeat units, 1 linker per 20-30 repeat units, 1 linker per 30-40 repeat units, 1 linker per 40-50 repeat units, 1 linker per 50-60 repeat units, or 1 linker per 60-70 repeat units. In some embodiments, a MenA polysaccharide is attached to the carrier protein through a linker as described above. In some embodiments, a MenC polysaccharide is attached to the carrier protein through a linker as described above.

In some embodiments, a polysaccharide conjugate composition according to the disclosure has improved stability relative to existing formulations. In some embodiments, stability is tested in terms of whether the free polysaccharide levels corresponding to each conjugated polysaccharide remain below 40% after a period of storage at 2°C-8°C, e.g., 2.5, 3, 3.5, 4, or 4.5 years. In some embodiments, stability is tested in terms of whether the free polysaccharide levels corresponding to each conjugated polysaccharide remain below 40% after a period of storage at 23°C-27°C, e.g., 2, 3, 4, 5, or 6 months. Certain quadrivalent MenACYW polysaccharide conjugate vaccines require lyophilization or other preservative measures at least in part as a result of low stability as liquid formulations with respect to one or more of the constituent conjugates. Lyophilization complicates both manufacturing and administration relative to a single liquid formulation. In some embodiments, a polysaccharide is attached to the carrier protein at multiple points. Multiple point attachment is generally a consequence of conjugation chemistry, e.g., periodate activation followed by reductive amination, or carbonyl diimidazole-based coupling (optionally with a linker) that can form a lattice of carrier protein and polysaccharide, together with appropriate polysaccharide size and loading ratio. For detailed discussion of such exemplary chemistry, see the Examples below. Exemplary polysaccharide sizes and loading ratios compatible with formation of a protein-polysaccharide lattice involving multiple points of attachment are at least 30 kDa (and exemplary size ranges discussed above) and polysaccharide/protein ratios of 0.3 to 1.5 (and exemplary loading ratio ranges discussed above). Without wishing to be bound by a particular theory, providing conjugates with multiple points of attachment between the polysaccharide and carrier protein may contribute to long-term stability of the conjugate in that multiple cleavage (e.g., hydrolytic) events would be needed to liberate polysaccharide fragments from the carrier protein. This contribution to long-term stability may be especially relevant to the MenA polysaccharide, which has phosphodiester linkages that may be more labile during storage in liquid than glycosidic bonds. In some embodiments, a composition comprises a MenA polysaccharide with multiple points of attachment to the carrier protein. In some embodiments, a composition comprises a MenC polysaccharide with multiple points of attachment to the carrier protein. In some embodiments, a composition comprises a MenY polysaccharide with multiple points of attachment to the carrier protein. In some embodiments, a composition comprises a MenW-135 polysaccharide with multiple points of attachment to the carrier protein. In some embodiments, a composition comprises MenA, MenC, MenY, and MenW-135 polysaccharides wherein each have multiple points of attachment to the carrier protein.

In some embodiments, a vaccine composition described herein is provided as a liquid formulation in a syringe, e.g., a pre-filled and/or silicone-free syringe. In some embodiments, such a syringe is commercially packaged for sale and/or distribution.

Further discussion of methods of producing conjugates as discussed above are described in WO2018/045286, which is incorporated herein by reference.

Formulation of the *Neisseria meningitidis* vaccine compositions for uses and methods disclosed herein can be accomplished using art recognized methods. The vaccine compositions/formulations of the present invention may also contain one or more adjuvants. Adjuvants include, by way of example and not limitation, aluminum adjuvants, Freund's Adjuvant, BAY, DC-chol, pcpp, monophoshoryl lipid A, CpG, QS-21, cholera toxin and formyl methionyl peptide. See, e.g., Vaccine Design, the Subunit and Adjuvant Approach, 1995 (M. F. Powell and M. J. Newman, eds., Plenum Press, N.Y.). The adjuvant, if present, can be an aluminum adjuvant, such as aluminum hydroxide or aluminum phosphate. In some embodiments, the vaccine compositions and formulations, e.g., the MenACWY-TT vaccine, does not comprise adjuvant. In some embodiments, the vaccine compositions and formulations, e.g., the MenACWY-TT vaccine comprises adjuvant.

The administration of the *Neisseria meningitidis* vaccine composition in methods and uses described herein can be as a single dose or as a part of a series, or as a booster after earlier administration of a *Neisseria meningitidis* vaccine, which may be the same *Neisseria meningitidis* vaccine composition or a different *Neisseria meningitidis* capsular saccharide conjugate vaccine. For example, a subject who received a dose early in life, can then be administered a booster dose of a *Neisseria meningitidis* vaccine composition described herein which is coadministered with an HPV L1 protein or proteins or an HPV vaccine as described herein, up to ten years later, e.g., at or after age 8. In some embodiments, a coadministration described herein occurs two months to ten years after a previously administered *Neisseria meningitidis* capsular saccharide conjugate vaccine, such as two to four months, four to six months, six to twelve months, 1 year to 2 years, 2 years to 3 years, 3 years to 4 years, 4 years to 5 years, 5 years to 6 years, 6 years to 7 years, 7 years to 8 years, 8 years to 9 years, or 9 years to 10 years after the previously administered *Neisseria meningitidis* capsular saccharide conjugate vaccine.

The booster dose will generate antibodies from primed B-cells, i.e., an anamnestic response. That is, the vaccine compositions and formulations, e.g., the MenACWY-TT vaccine, elicits a high primary (i.e., following a single administration of vaccine) functional antibody response in younger and older populations, and is capable of eliciting an anamnestic response (i.e., following a booster administration), demonstrating that the protective immune response elicited by the vaccine compositions and formulations, e.g., the MenACWY-TT vaccine of the present invention is long-lived.

In some embodiments, the administration of the *Neisseria meningitidis* vaccine is via intramuscular injection. In other embodiments, the administration is subcutaneous, intradermal, intraperitoneal, parenteral or intravenous. Compositions and formulations may be in admixture with a suitable carrier, diluent, or excipient such as a sodium acetate buffered saline solution, sterile water, physiological saline or the like. The compositions/formulations can also be lyophilized. The compositions/formulations can contain auxiliary substances such as wetting or emulsifying agents, pH buffering agents, gelling or viscosity enhancing additives, preservatives, and the like, depending upon the route of administration and the preparation desired. Standard texts, such as "REMINGTON'S PHARMACEUTICAL SCIENCE", 17th edition, 1985, incorporated herein by reference, may be consulted to prepare suitable preparations, without undue experimentation.

In some embodiments, the vaccine composition/formulation is a liquid preparation. In some embodiments, the vaccine composition/formulation, e.g., MenACWY-TT vaccine, is a liquid composition to be administered by injection to animals, children, particularly small children, older adults, e.g., over 55, 60, 65, 70, 75, 80, or 90.

The choice of suitable carriers and other additives will depend on the exact route of administration and the nature of the particular dosage form.

In one embodiment, the vaccine compositions and formulations, e.g., the MenACYW-TT vaccine, comprises a pharmaceutically acceptable preservative, carrier, buffer excipient, or the like. In one embodiment, the pharmaceutically acceptable preservative, carrier, or excipient increases or extends the shelf life of the compositions. In some embodiments, the vaccine comprises a buffer. In some embodiments, the buffer is sodium acetate. In some embodiments, the buffer is sodium phosphate. In some embodiments, the buffer is present at a concentration ranging from 10 mM to 100 mM, for example, 10 mM to 70 mM, 15 mM to 45 mM, 20 mM to 40 mM, 40 mM to 60 mM, or 60 mM to 100 mM. In some embodiments, the buffer has a pH of 4.5 to 7.5, 4.5 to 7.0, 4.5 to 6.5, 4.5 to 6.0, 4.5 to 5.5, or 4.5 to 5.0. In some embodiments, the buffer has a pH ranging from 5.5 to 7.0, for example, 5.75 to 6.25, or 6.25 to 6.75. In some embodiments, the buffer has a pH of 5.5 to 6.5. In some embodiments, the buffer has a pH of 5 or 6. In some embodiments, the vaccine composition comprises a pharmaceutically acceptable salt. In some embodiments, the vaccine composition/formulation comprises saline. In some embodiments, the saline comprises or is NaCl. The NaCl may be present at a concentration of 0.45% to 0.9% w/v, such as 0.5% to 0.85% w/v, or 0.6% to 0.8% w/v, or 0.6%, 0.67%, 0.75%, 0.8%, 0.85%, or 0.9%.

In one embodiment, each component of the composition is chemically inert with respect to the *N. meningitidis* polysaccharide-protein carrier conjugates.

In some embodiments, the vaccine compositions and formulations, e.g., the MenACWY-TT vaccine, are formulated as a single unit dose. In some embodiments, the single unit dose comprises from 6 µg to 15 µg of each of the MenA, MenC, MenW-135, and MenY polysaccharides. In some embodiments, the single unit dose comprises 4, 5, 6, 7, 8, 9, 10, 11, 12, 13, 14, or 15 µg of each of the MenA, MenC, MenW-135, and MenY polysaccharides. In some embodiments, the carrier protein is present in an amount from 50 µg to 80 µg in the single unit dose. In some embodiments, the carrier protein is present in an amount from 45, 50, 55, 60, 65, 70, 75, or 80 µg in the single unit dose.

In some embodiments, the vaccine compositions and formulations, e.g., the MenACWY-TT vaccine is formulated as a 0.5 mL dose in sodium acetate, sodium acetate buffered saline or similar buffer. In some embodiments, the 0.5 mL dose comprises 2, 3, 4, 5, 6, 7, 8, 9, 10, 11, 12, 13, 14, or 15 µg each of serogroups A, C, Y, and W-135 conjugated to 50, 55, 60, 65, 70, 80, 85, or 90 µg of tetanus toxoid protein. In some embodiments, this 0.5 mL dose is administered intramuscularly.

### 2. Exemplary HPV L1 proteins and vaccines for uses disclosed herein

In some embodiments, the method of immunization comprises coadministering one or more HPV L1 proteins with a *Neisseria meningitidis* vaccine composition described herein. In some embodiments, the method of immunization comprises coadministering an HPV vaccine comprising one or more HPV L1 proteins with a *Neisseria meningitidis* vaccine composition described herein. HPV L1 protein is the major capsid protein of HPV and has been found to be capable of use as a vaccine against HPV, e.g., in Gardasil™.

In some embodiments, the method of immunization comprises coadministering an HPV Type 18 L1 protein with the *Neisseria meningitidis* vaccine composition. In some embodiments, the method of immunization comprises coadministering an HPV Type 16 L1 protein with the *Neisseria meningitidis* vaccine composition. In some embodiments, the method of immunization comprises coadministering an HPV Type 11 L1 protein with the *Neisseria meningitidis* vaccine composition. In some embodiments, the method of immunization comprises coadministering an HPV Type 6 L1 protein with the *Neisseria meningitidis* vaccine composition. In some embodiments, two, three, or four of the foregoing HPV L1 proteins are coadministered, e.g., Type 16 and 18 L1 proteins. In some embodiments, HPV Type 6, 11, 16, and 18 L1 proteins. Where more than one HPV protein is administered, they may be provided in the same composition, e.g., as in Cervarix™ or Gardasil™. In some embodiments, one or more (e.g., each) of the HPV L1 proteins are provided in the form of virus-like particles (VLPs). *See, e.g.,* US Patent No. 5,820,870 for disclosure of virus-like particles comprising an HPV L1 protein. Further discussion of HPV virus-like particles and Gardasil™ is provided in Shi et al., Clin. Pharmacol. Ther. (2007) 81, 259-64, and references cited therein.

In some embodiments, the HPV Type 6 L1 protein (e.g., as a VLP) is administered at a dose of 20 µg. In some embodiments, the HPV Type 11 L1 protein (e.g., as a VLP) is administered at a dose of 40 µg. In some embodiments, the HPV Type 16 L1 protein (e.g., as a VLP) is administered at a dose of 40 µg. In some embodiments, the HPV Type 18 L1 protein (e.g., as a VLP) is administered at a dose of 20 µg.

In some embodiments, one or more (e.g., each) of the HPV L1 proteins, which may be at the dosages discussed above, are administered in a pharmaceutical composition. In some embodiments, the pharmaceutical composition comprises amorphous aluminum hydroxyphosphate sulfate adjuvant. In some embodiments, the pharmaceutical composition comprises sodium chloride. In some embodiments, the pharmaceutical composition comprises L-histidine. In some embodiments, the pharmaceutical composition comprises polysorbate 80. In some embodiments, the pharmaceutical composition comprises sodium borate. In some embodiments, the pharmaceutical composition comprises any combination, or all, of the foregoing ingredients. In some embodiments, the *Neisseria meningitidis* vaccine composition is coadministered with quadrivalent human papillomavirus [type 6, 11, 16, 18] (HPV) recombinant vaccine, i.e., Gardasil™.

### 3. Exemplary Uses and Methods of Immunization in which a Neisseria meningitidis vaccine composition and an HPV vaccine or HPV L1 protein are coadminstered

### a) Absence of interference with the development of immunity

As noted above, *Neisseria meningitidis* vaccine compositions described herein can be coadministered with HPV L1 proteins, e.g., as in Gardasil™, without interference with the development of immunity against HPV and/or Neisseria meningitidis.
Accordingly, in some embodiments, the administration of the *Neisseria meningitidis* vaccine composition does not interfere with the development of immunity against HPV Type 18. In some embodiments, the administration of the *Neisseria meningitidis* vaccine composition does not interfere with the development of immunity against HPV Type 16. In some embodiments, the administration of the *Neisseria meningitidis* vaccine composition does not interfere with the development of immunity against HPV Type 11. In some embodiments, the administration of the *Neisseria meningitidis* vaccine composition does not interfere with the development of immunity against HPV Type 6. In some embodiments, the administration of the *Neisseria meningitidis* vaccine composition does not interfere with the development of immunity against HPV Type 18 and HPV Type 16. In some embodiments, the administration of the *Neisseria meningitidis* vaccine composition does not interfere with the development of immunity against HPV Type 18, HPV Type 16, HPV Type 11, and HPV Type 6.

In some embodiments, the administration of the HPV protein or proteins does not interfere with the development of immunity against *Neisseria meningitidis* serogroup A. In some embodiments, the administration of the HPV protein or proteins does not interfere with the development of immunity against *Neisseria meningitidis* serogroup C. In some embodiments, the administration of the HPV protein or proteins does not interfere with the development of immunity against *Neisseria meningitidis* serogroup Y. In some embodiments, the administration of the HPV protein or proteins does not interfere with the development of immunity against *Neisseria meningitidis* serogroup W-135. In some embodiments, the administration of the HPV protein or proteins does not interfere with the development of immunity against *Neisseria meningitidis* serogroups A, C, Y, and W-135.

Interference with the development of immunity as in any of the foregoing embodiments may be determined by comparing geometric mean titers (GMTs).

As an additional or alternative feature to non-interference, in some embodiments, coadministration of the *Neisseria meningitidis* vaccine composition with the HPV protein or proteins gives a GMT against an HPV Type that is within the 95% confidence interval of the GMT against the HPV Type resulting from administration of the HPV protein or proteins without the *Neisseria meningitidis* vaccine composition. For example, in some embodiments, coadministration of the *Neisseria meningitidis* vaccine composition with the HPV protein or proteins gives a GMT against HPV Type 18 that is within the 95% confidence interval of the GMT against HPV Type 18 resulting from administration of the HPV protein or proteins without the *Neisseria meningitidis* vaccine composition. In another example, coadministration of the *Neisseria meningitidis* vaccine composition with the HPV protein or proteins gives a GMT against HPV Type 16 that is within the 95% confidence interval of the GMT against HPV Type 16 resulting from administration of the HPV protein or proteins without the *Neisseria meningitidis* vaccine composition. In another example, coadministration of the *Neisseria meningitidis* vaccine composition with the HPV protein or proteins gives a GMT against HPV Type 11 that is within the 95% confidence interval of the GMT against HPV Type 11 resulting from administration of the HPV protein or proteins without the *Neisseria meningitidis* vaccine composition. In another example, coadministration of the *Neisseria meningitidis* vaccine composition with the HPV protein or proteins gives a GMT against HPV Type 6 that is within the 95% confidence interval of the GMT against HPV Type 6 resulting from administration of the HPV protein or proteins without the *Neisseria meningitidis* vaccine composition. In some embodiments, the foregoing is true for HPV Types 16 and 18. In some embodiments, the foregoing is true for HPV Types 6, 11, 16 and 18.

As another additional or alternative feature to non-interference, in some embodiments, coadministration of the *Neisseria meningitidis* vaccine composition with the HPV protein or proteins gives a GMT against a *Neisseria meningitidis* serogroup that is within the 95% confidence interval of the GMT against the serogroup resulting from administration of the *Neisseria meningitidis* vaccine composition without the HPV protein or proteins. In some embodiments, the serogroup is serogroup A. In some embodiments, the serogroup is serogroup C. In some embodiments, the serogroup is serogroup W-135. In some embodiments, the serogroup is serogroup Y. In some embodiments, the foregoing is true for at least two or three serogroups. In some embodiments, the foregoing is true for serogroups A, C, W-135, and Y.

### b) Routes and sites of administration of coadministered compositions

In some embodiments, the HPV protein or proteins are administered by injection. In some embodiments, the *Neisseria meningitidis* vaccine composition is administered by injection. Injections may be performed using a silicone-free syringe. In some embodiments, the HPV protein or proteins are administered intramuscularly. In some embodiments, the *Neisseria meningitidis* vaccine composition is administered intramuscularly. In some embodiments, the HPV protein or proteins are administered to an anatomical location different from that to which the *Neisseria meningitidis* vaccine composition is administered (e.g., first and second different intramuscular locations). For example, the HPV protein or proteins and the *Neisseria meningitidis* vaccine composition can be administered to opposite shoulders/deltoid muscles. Administration may also be to different quadriceps muscles, or to a deltoid and a quadriceps.

### c) Additional vaccines

In some embodiments, an additional vaccine is coadministered with the *Neisseria meningitidis* vaccine composition, such as a vaccine for one or more of diphtheria, pertussis, or tetanus. In some embodiments, a diphtheria-tetanus-pertussis vaccine is coadministered. Exemplary diphtheria-tetanus-pertussis vaccines are Tetanus, diphtheria, acellular pertussis [Tdap] vaccine or DTaP5. The additional vaccine can be administered to the same anatomical location (e.g., right or left shoulder/deltoid muscle) as the HPV protein or proteins. In some embodiments, the additional vaccine (e.g., diphtheria-tetanus-pertussis vaccine) does not interfere with the development of immunity against one or more *Neisseria meningitidis* serogroups, such as serogroups A, C, W-135, and/or Y.

### d) Coadministration Dosing Regimens

The HPV protein or proteins may be administered in a two- or three-dose schedule. The coadministration of the *Neisseria meningitidis* vaccine composition may be with any dose of the HPV protein or proteins. In some embodiments, the coadministration of the *Neisseria meningitidis* vaccine composition is with the first dose of the HPV protein or proteins. In some embodiments, the subject has not previously received a dose of an HPV vaccine.

In some embodiments, the subject has not previously received a dose of the *Neisseria meningitidis* vaccine composition. In some embodiments, the subject has not previously received a dose of a *Neisseria meningitidis* vaccine against serogroup A. In some embodiments, the subject has not previously received a dose of a *Neisseria meningitidis* vaccine against serogroup C. In some embodiments, the subject has not previously received a dose of a *Neisseria meningitidis* vaccine against serogroup W-135. In some embodiments, the subject has not previously received a dose of a *Neisseria meningitidis* vaccine against serogroup Y. In some embodiments, the subject has not previously received a dose of a *Neisseria meningitidis* vaccine against any of serogroups A, C, W-135, or Y.

### e) Subjects

In some embodiments, the subject is a male or female aged at least 8 years. In some embodiments, the subject is a male or female aged 8 to 25 years. In some embodiments, the subject is a male or female aged 9 to 25 years. In some embodiments, the subject is a male or female aged 10 to 25 years. In some embodiments, the subject is a male or female aged 8 to 21 years. In some embodiments, the subject is a male or female aged 9 to 21 years. In some embodiments, the subject is a male or female aged 10 to 21 years. In some embodiments, the subject is a male or female aged 8 to 20 years. In some embodiments, the subject is a male or female aged 9 to 20 years. In some embodiments, the subject is a male or female aged 10 to 20 years. In some embodiments, the subject is a male or female aged 8 to 19 years. In some embodiments, the subject is a male or female aged 9 to 19 years. In some embodiments, the subject is a male or female aged 10 to 19 years. In some embodiments, the subject is a male or female aged 8 to 18 years. In some embodiments, the subject is a male or female aged 9 to 18 years. In some embodiments, the subject is a male or female aged 10 to 18 years. In some embodiments, the subject is a male or female aged 8 to 17 years. In some embodiments, the subject is a male or female aged 9 to 17 years. In some embodiments, the subject is a male or female aged 10 to 17 years. In some embodiments, the subject is a male or female aged 8 to 14 years. In some embodiments, the subject is a male or female aged 9 to 14 years. In some embodiments, the subject is a male or female aged 10 to 14 years.

In some embodiments, the coadministration of the *Neisseria meningitidis* vaccine composition and the HPV protein or proteins is effective in males and females, i.e., regardless of the biological sex of the subject.

### f) Exemplary embodiments of methods and uses

The following items represent exemplary disclosed method embodiments. The corresponding *Neisseria meningitidis* vaccine compositions for use in such methods are also disclosed as if explicitly set forth herein.
Item 1 is a method of immunization against *Neisseria meningitidis* serogroups A, C, Y, and W-135 and human papilloma virus, the method comprising coadministering a *Neisseria meningitidis* vaccine composition and an HPV Type 18 L1 protein to a subject in need thereof, wherein:
   the *Neisseria meningitidis* vaccine composition comprises
      a) a first conjugate of MenA capsular polysaccharide to tetanus toxoid;
      b) a second conjugate of MenC capsular polysaccharide to tetanus toxoid;
      c) a third conjugate of MenW-135 capsular polysaccharide to tetanus toxoid; and
      d) a fourth conjugate of MenY capsular polysaccharide to tetanus toxoid;
   the second conjugate is a population comprising double-end-linked conjugated polysaccharides and single-end-linked conjugated polysaccharides which both are attached to the tetanus toxoid through a secondary amine, and the polysaccharides of the second conjugate have an O-acetylation level of 0.3 µmol/mg polysaccharide to 1.6 µmol/mg polysaccharide.
Item 2 is a method of immunization against *Neisseria meningitidis* serogroups A, C, Y, and W-135 and human papilloma virus, the method comprising coadministering a *Neisseria meningitidis* vaccine composition and an HPV Type 18 L1 protein to a subject in need thereof, wherein:
   the *Neisseria meningitidis* vaccine composition comprises
      a) a first conjugate of MenA capsular polysaccharide to tetanus toxoid;
      b) a second conjugate of MenC capsular polysaccharide to tetanus toxoid;
      c) a third conjugate of MenW-135 capsular polysaccharide to tetanus toxoid; and
      d) a fourth conjugate of MenY capsular polysaccharide to tetanus toxoid;
   the second conjugate is a population comprising single-end-linked conjugated polysaccharides which are attached to the tetanus toxoid through a secondary amine, wherein the single-end-linked conjugated polysaccharides have a terminal unlinked saccharide, wherein the terminal saccharide has a primary hydroxyl or secondary amine linkage at the 7 position, or wherein the reducing end is modified with a (2-hydroxy)ethoxy or secondary amine linkage.
Item 3 is a method of immunization against *Neisseria meningitidis* serogroups A, C, Y, and W-135 and human papilloma virus, the method comprising coadministering a *Neisseria meningitidis* vaccine composition and an HPV Type 18 L1 protein to a subject in need thereof, wherein:
   the *Neisseria meningitidis* vaccine composition comprises
      a) a first conjugate of MenA capsular polysaccharide to tetanus toxoid;
      b) a second conjugate of MenC capsular polysaccharide to tetanus toxoid;
      c) a third conjugate of MenW-135 capsular polysaccharide to tetanus toxoid; and
      d) a fourth conjugate of MenY capsular polysaccharide to tetanus toxoid;
   the MenA capsular polysaccharide is attached to the tetanus toxoid through a linker comprising a carbamate, a spacer, and an amide, wherein the spacer is between the carbamate and the amide and comprises 2-10 linear carbons, and the first conjugate has a polysaccharide to tetanus toxoid mass ratio of 0.3 to 1.5.
Item 4 is a method of immunization against *Neisseria meningitidis* serogroups A, C, Y, and W-135 and human papilloma virus, the method comprising coadministering a *Neisseria meningitidis* vaccine composition and an HPV Type 18 L1 protein to a subject in need thereof, wherein:
   the *Neisseria meningitidis* vaccine composition comprises
      a) a first conjugate of MenA capsular polysaccharide to tetanus toxoid;
      b) a second conjugate of MenC capsular polysaccharide to tetanus toxoid;
      c) a third conjugate of MenW-135 capsular polysaccharide to tetanus toxoid; and
      d) a fourth conjugate of MenY capsular polysaccharide to tetanus toxoid;
   the MenA capsular polysaccharide is attached to the tetanus toxoid through a linker comprising a carbamate, a spacer, and an amide, wherein the spacer is between the carbamate and the amide and comprises 2-10 linear carbons; and wherein the MenC, MenW-135, and MenY capsular polysaccharides are attached to the tetanus toxoid through a secondary amine; and
   at least one of the conjugates has a weight average molecular weight ranging from 300 kDa to 1500 kDa.
Item 5 is a method of immunization against *Neisseria meningitidis* serogroups A, C, Y, and W-135 and human papilloma virus, the method comprising coadministering a *Neisseria meningitidis* vaccine composition and an HPV Type 18 L1 protein to a subject in need thereof, wherein:
   the *Neisseria meningitidis* vaccine composition comprises
      a) a first conjugate of MenA capsular polysaccharide to tetanus toxoid;
      b) a second conjugate of MenC capsular polysaccharide to tetanus toxoid;
      c) a third conjugate of MenW-135 capsular polysaccharide to tetanus toxoid; and
      d) a fourth conjugate of MenY capsular polysaccharide to tetanus toxoid;
   one or more of the first, second, third, and fourth conjugates has a weight average molecular weight ranging from 300 kDa to 1500 kDa; and the composition comprises less than 20% free polysaccharide by weight relative to total polysaccharide.
Item 6 is a method of immunization against *Neisseria meningitidis* serogroups A, C, Y, and W-135 and human papilloma virus, the method comprising coadministering a *Neisseria meningitidis* vaccine composition and an HPV Type 18 L1 protein to a subject in need thereof, wherein:
   the *Neisseria meningitidis* vaccine composition comprises
      a) a first conjugate of MenA capsular polysaccharide to tetanus toxoid;
      b) a second conjugate of MenC capsular polysaccharide to tetanus toxoid;
      c) a third conjugate of MenW-135 capsular polysaccharide to tetanus toxoid; and
      d) a fourth conjugate of MenY capsular polysaccharide to tetanus toxoid;
   one or more of the first, second, third, and fourth conjugates have a polysaccharide to tetanus toxoid mass ratio of 0.3 to 1.5; and the composition comprises less than 20% free polysaccharide by weight relative to total polysaccharide.
Item 7 is the method of any one of the preceding items, wherein the first, second, third, and/or fourth conjugates are a population comprising molecules with a molecular weight in the range of 700 kDa to 1400 kDa or 800 kDa to 1300 kDa.
Item 8 is the method of any one of items 4, 5, or 7, wherein molecular weight is determined by multi-angle light scattering (MALS).
Item 9 is the method of any one of the preceding items, wherein the administration of the *Neisseria meningitidis* vaccine composition does not interfere with the development of immunity against HPV Type 18.
Item 10 is the method of any one of the preceding items, wherein the method further comprises coadministering an HPV Type 16 L1 protein with the *Neisseria meningitidis* vaccine composition.
Item 11 is the method of any one of the preceding items, wherein the administration of the *Neisseria meningitidis* vaccine composition does not interfere with the development of immunity against HPV Type 16.
Item 12 is the method of any one of the preceding items, wherein the method further comprises coadministering an HPV Type 6 L1 protein and/or an HPV Type 11 L1 protein with the *Neisseria meningitidis* vaccine composition.
Item 13 is the method of item 12, wherein the administration of the *Neisseria meningitidis* vaccine composition does not interfere with the development of immunity against HPV Type 6 and/or HPV Type 11.
Item 14 is the method of any one of the preceding items, wherein the administration of the HPV protein or proteins does not interfere with the development of immunity against *Neisseria meningitidis* serogroups A, C, Y, and/or W-135.
Item 15 is the method of any one of the preceding items, wherein interference or non-interference with the development of immunity against HPV Type 18, 16, 11, and/or 6 and/or *Neisseria meningitidis* serogroups A, C, Y, and/or W-135 is determined by comparing geometric mean titers.
Item 16 is the method of any one of the preceding items, wherein the coadministration of the *Neisseria meningitidis* vaccine composition and the HPV protein or proteins does not result in increased risk of injection site swelling relative to administration of the HPV protein or proteins without the *Neisseria meningitidis* vaccine composition.
Item 17 is the method of any one of the preceding items, wherein the method further comprises coadministering a diphtheria-tetanus-pertussis vaccine with the *Neisseria meningitidis* vaccine composition, optionally wherein the diphtheria-tetanus-pertussis vaccine is Tetanus, diphtheria, acellular pertussis [Tdap] vaccine or DTaP5
Item 18 is The *Neisseria meningitidis* vaccine composition for use according to any one of the preceding items, wherein the administration of the diphtheria-tetanus-pertussis vaccine does not interfere with the development of immunity against *Neisseria meningitidis* serogroups A, C, Y, and/or W-135.
Item 19 is the method of any one of the preceding items, wherein the subject is a male or female aged from 9 to 18 years, from 10 to 17 years, from 11 to 18 years, or from 9 to 14 years.
Item 20 is the method of any one of the preceding items, wherein the the coadministration of the *Neisseria meningitidis* vaccine composition is with the first dose of the HPV protein or proteins, and/or wherein the the HPV protein or proteins are administered as part of a series of three doses.
Item 21 is the method of any one of the preceding items, wherein the *Neisseria meningitidis* vaccine composition and the HPV protein or proteins are administered intramuscularly and/or to different sites.
Item 22 is the method of any one of the preceding items, wherein the MenC capsular polysaccharide has a degree of O-acetylation ranging from 0.6 to 1.5 µmol/mg polysaccharide or 0.8 to 1.4 µmol/mg polysaccharide.
Item 23 is the method of any one of the preceding items, wherein the degree of O-acetylation is greater than or equal to 0.7, 0.8, 0.9, 1.0, 1.1, or 1.2 µmol/mg polysaccharide.
Item 24 is the method of any one of the preceding items, wherein the degree of O-acetylation is less than or equal to 0.8, 0.9, 1.0, 1.1, 1.2, 1.3, or 1.4 µmol/mg polysaccharide.
Item 25 is the method of any one of the preceding items, wherein the conjugate comprising MenC polysaccharide is a population comprising double-end-linked conjugated polysaccharides and single-end-linked conjugated polysaccharides.
Item 26 is the method of item 25, wherein the single-end-linked polysaccharides of the second conjugate comprise a terminal unlinked saccharide, wherein the single-end-linked conjugated polysaccharides have a terminal unlinked saccharide, wherein the terminal saccharide has a primary hydroxyl at the 7 position, or wherein the reducing end is modified with a (2-hydroxy)ethoxy.
Item 27 is the method of any one of the preceding items, wherein the conjugate comprising MenC polysaccharide comprises one or more modifications chosen from (i) a primary hydroxyl at the 7 position, (ii) a (2-hydroxy)ethoxy at the reducing end, and (iii) a conjugation to the carrier protein, wherein the modifications are present at no less than 25 nmol/mg polysaccharide.
Item 28 is the method of any one of the preceding items, comprising a conjugate of MenW-135 and/or MenY polysaccharide which comprises one or more modifications chosen from (i) a primary hydroxyl at a position of a vicinal diol in a native MenW-135 or MenY polysaccharide and (ii) a conjugation to the carrier protein, wherein the modifications are present at no less than 60 nmol/mg polysaccharide.
Item 29 is the method of item 27 or 28, wherein the modifications are present in an amount less than 200 nmol/mg polysaccharide, less than 150 nmol/mg polysaccharide, less than 150 nmol/mg polysaccharide, less than 100 nmol/mg polysaccharide, or less than 80 nmol/mg polysaccharide.
Item 30 is the method of any one of the preceding items, wherein the MenC polysaccharide is reduced in size by 3x-8x relative to native MenC polysaccharide.
Item 31 is the method of any one of the preceding items, comprising a conjugate of MenA capsular polysaccharide to a carrier protein having a polysaccharide to carrier protein mass ratio of 0.5 to 1.5 or 0.7 to 1.4.
Item 32 is the method of item 31, wherein the MenA conjugate has a polysaccharide to carrier protein mass ratio of 0.8 to 1.3.
Item 33 is the method of any one of the preceding items, comprising a conjugate of MenC and/or MenY capsular polysaccharide to a carrier protein having a polysaccharide to carrier protein mass ratio of 0.3 to 1.1.
Item 34 is the method of item 33, wherein the MenC conjugate has a polysaccharide to carrier protein mass ratio of 0.4 to 0.8.
Item 35 is the method of any one of the preceding items, comprising a conjugate of MenW-135 capsular polysaccharide to a carrier protein having a polysaccharide to carrier protein mass ratio of 0.3 to 1.3.
Item 36 is the method of item 35, wherein the MenW-135 conjugate has a polysaccharide to carrier protein mass ratio of 0.6 to 1.3.
Item 37 is the method any one of the preceding items, comprising a conjugate of MenY capsular polysaccharide to a carrier protein having a polysaccharide to carrier protein mass ratio of 0.5 to 1.3.
Item 38 is the method of item 37, wherein the MenY conjugate has a polysaccharide to carrier protein mass ratio of 0.5 to 0.9.
Item 39 is the method of any one of the preceding items, wherein the composition comprises less than 20% free polysaccharide by weight.
Item 40 is the method of item 39, wherein the composition comprises less than 10% free polysaccharide by weight, less than 5% free polysaccharide by weight, or substantially lacks free polysaccharide.
Item 41 is the method of any one of the preceding items, wherein the polysaccharide of the MenA, MenC, MenW-135, or MenY conjugate is attached to the carrier protein through a linker.
Item 42 is the method of item 41, wherein the linker comprises 2-10 linear carbons.
Item 43 is the method of items 41 or 42, wherein the linker is present in the MenA, MenC, MenW-135, or MenY conjugate at a ratio of one linker per 10-100 saccharide repeat units.
Item 44 is the method of items 41 or 42, wherein the linker is present in the MenA, MenC, MenW-135, or MenY conjugate at a ratio of one linker per 20-60 saccharide repeat units.
Item 45 is the method of items 41 or 42, wherein the linker comprises a spacer between a first carbonyl and a second carbonyl, and the spacer comprises 4-8 carbons.
Item 46 is the method of any one of items 41-45, wherein the linker of the MenA conjugate comprises a residue of a dihydrazide.
Item 47 is the method of item 46, wherein the linker of the MenA conjugate comprises a residue of adipic acid dihydrazide.
Item 48 is the method of any one of the preceding items, wherein the polysaccharide of the MenA, MenC, MenW-135, and/or MenY conjugate is attached to the carrier protein through a linker of formula I: 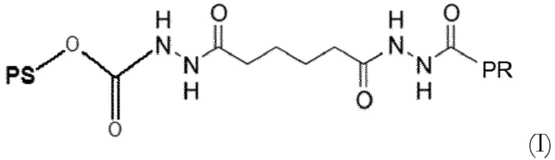 wherein PS indicates attachment to the polysaccharide and PR indicates attachment to the carrier protein.
Item 49 is the method of any one of items 41-48, wherein the linker is in the MenA conjugate.
Item 50 is the method of any one of items 41-48, wherein the linker is in the MenC conjugate.
Item 51 is the method of any one of items 41-48, wherein the linker is in the MenW-135 conjugate.
Item 52 is the method of any one of items 41-48, wherein the linker is in the MenY conjugate.
Item 53 is the method of any one of the preceding items, wherein the polysaccharide of the MenA, MenC, MenW-135, and/or MenY conjugate is attached to the carrier protein as shown in formula II:
   PR-NH-CH₂-PS (II)
   wherein PS indicates attachment to the polysaccharide and PR indicates attachment to the carrier protein.
Item 54 is the method of item 53, wherein the polysaccharide of the MenA conjugate is attached to the carrier protein as shown in formula II.
Item 55 is the method of item 53, wherein the polysaccharide of the MenC conjugate is attached to the carrier protein as shown in formula II.
Item 56 is the method of item 53, wherein the polysaccharide of the MenW-135 conjugate is attached to the carrier protein as shown in formula II.
Item 57 is the method of item 53, wherein the polysaccharide of the MenY conjugate is attached to the carrier protein as shown in formula II.
Item 58 is the method of any one of the preceding items, wherein the *Neisseria meningitidis* vaccine composition is free of adjuvant.
Item 59 is the method of any one of the preceding items, wherein the *Neisseria meningitidis* vaccine composition further comprises a pharmaceutically acceptable buffer.
Item 60 is the method of item 59, comprising acetate buffer with a pH of 5.5 to 6.5.
Item 61 is the method of any one of the preceding items, wherein the *Neisseria meningitidis* vaccine composition further comprises further comprising a pharmaceutically acceptable salt.
Item 62 is the method of item 61, wherein the pharmaceutically acceptable salt is sodium chloride.
Item 63 is the method of item 61 or 62, wherein the pharmaceutically acceptable salt is present at 0.45% to 0.9% w/v, or 0.5% w/v to 0.85% w/v.
Item 64 is the method of any one of the preceding items, wherein at least one, two, three, or all four of the first, second, third, and fourth conjugates comprise multiple points of attachment between the polysaccharides and the carrier proteins.
Item 65 is the method of any one of the preceding items, wherein the *Neisseria meningitidis* vaccine composition is administered as a dose comprising from 6 µg to 15 µg or 4 µg to 10 µg of each of the MenA, MenC, MenW-135, and MenY polysaccharides.
Item 66 is the method of item 65, wherein the tetanus toxoid is present in an amount from 50 µg to 80 µg.
Item 67 is the method of any one of the preceding items, wherein *Neisseria meningitidis* vaccine composition is administered using a silicone-free syringe.
Item 68 is the method of any one of the preceding items, wherein the vaccine is administered intramuscularly.
Item 69 is the method of any one of the preceding items, wherein the vaccine is administered as a 0.5 mL dose.
Item 70 is the method of any one of the preceding items, wherein the subject previously received a *Neisseria meningitidis* capsular saccharide conjugate vaccine.
Item 71 is the method of item 70, wherein the subject received the *Neisseria meningitidis* capsular saccharide conjugate vaccine four months to ten years earlier.
Item 72 is the method of any one of items 1-69, wherein the subject did not previously receive a *Neisseria meningitidis* capsular saccharide conjugate vaccine.
Item 73 is the method of any one of the preceding items, wherein the subject did not previously receive an HPV vaccine.

### IV. EXAMPLES

The following are examples of methods, uses, and compositions disclosed herein. It is understood that various other embodiments may be practiced, given the general and detailed descriptions provided above. The following examples are given for the purpose of illustrating the present teachings and shall not be construed as being a limitation on the scope of the disclosure or claims.

### 1. Preparation of Group A Conjugates

### Example 1A

Group A purified capsular polysaccharide is dissolved in 10% by weight tetrabutylammonium chloride (TBAC) in dimethylsulfoxide (DMSO) to a target concentration of 8 mg/mL. The solution is mixed until the polysaccharide is fully dissolved at 19 - 25°C. The dissolved polysaccharide is activated by addition of a target concentration of 35-45 molar excess of carbonyldiimidazole (CDI) per N-acetylmannosamine phosphate repeat unit (PS RU), and mixed for 50 to 70 minutes at 19 - 25°C (FIG. 1C, first reaction; product shown in FIG. 1E). The polysaccharide solution is diluted 1:2 with WFI (50% v/v) to adjust the concentration of the activated polysaccharide to 4 mg/mL in 50% DMSO. The solution is derivatized by adding Adipic acid Dihydrazide (ADH) (1.0 mol ADH per 1-3 mol PS RU) (FIG. 1C, second reaction; product shown in FIG. 1F) and mixed overnight at room temperature. The reaction gives an amount of derivatization such that there is one bound ADH per 10 to 100 polysaccharide repeat units, e.g., one bound ADH per 20, 30, 40, 50, or 60 polysaccharide repeat units. The activated polysaccharide is concentrated by ultrafiltration on the 10kDa MWCO PES membrane and then diafiltered against 12 - 18 volume exchanges of physiological saline. The target concentration is approximately 30mg/mL. The activated polysaccharide is filtered and is stored at 1-5°C.

Purified Tetanus Toxoid protein (TT) is filtered through a 0.2 micron membrane and stored at 1-5°C. The derivatized polysaccharide and concentrated Tetanus protein are mixed together, in a ratio of 0.5:1, 1:1, 2:1, 3:1, 4:1, or 5:1. An aliquot of 100 mg/mL of the cross linking agent 1-ethyl-3-(dimethylaminopropyl) carbodiimide (EDAC) in 1.0 M MES buffer, pH 5.7 is added to the polysaccharide-protein mixture such that a final concentration of EDAC is 10 mg/mL and MES is 100 mM. Saline is added to give target concentrations of 16 mg/mL Polysaccharide and 4 mg/mL TT. The final pH is adjusted to 5.5 - 5.9 and the reaction is mixed at 15.6 - 23.9°C for 16-24 hours. During this time, the EDAC and TT react to form an O-acylisourea intermediate (FIG. 1B). The O-acylisourea intermediate and the derivatized polysaccharide then form a conjugate (FIG. 1G; products shown in FIG. 1H and FIG. 1A).

Ammonium sulfate is added to the conjugate reaction to yield a 1 M ammonium sulfate concentration. The pH is adjusted to 7 and is mixed until dissolved at room temperature. The conjugate reaction mixture is applied to a HIC column packed with phenyl resin. The unconjugated polysaccharide is eluted with 2 to 7 column volumes of 1 M ammonium sulfate solution. The conjugate is eluted with WFI. In this and subsequent examples, the HIC purification of the conjugate can provide a product in which less than 20% of the polysaccharide by mass is free (unconjugated) polysaccharide. The conjugate eluate is diafiltered against 10 volume exchanges of 50mM sodium acetate, pH 6.0, using a 100 kDa MWCO PES membrane. The final filtration of the purified conjugate is performed using a 0.2 micron membrane and the conjugate is stored at 1 - 5°C.

### Example 1B

Group A purified capsular polysaccharide is dissolved in tetrabutylammonium chloride (TBAC)/dimethylsulfoxide (DMSO) by weight to a target concentration of 6 mg/mL. The solution is mixed for 16 to 24 hours at 19 - 25°C. The dissolved polysaccharide is activated by addition of a target concentration of 35-45 molar excess of carbonyldiimidazole (CDI) per N-acetylmannosamine phosphate repeat unit (PS RU), and mixed for 50 to 70 minutes at 19 - 25°C (FIG. 1C, first reaction; product shown in FIG. 1E). The polysaccharide solution is diluted 1:2 with WFI (45 - 55% v/v) such that the activated polysaccharide is at a target concentration of 3 mg/mL in 50% DMSO.

Purified Tetanus Toxoid protein (TT) is filtered through a 0.2 micron membrane and stored at 1-5°C. The Tetanus protein is added to a final concentration of 1 mg/mL. During this time, the activated polysaccharide and TT react to form a conjugate with a carbamate linkage (FIG. 1D). The reaction proceeds overnight at room temperature.

Ammonium sulfate is added to the conjugate reaction to yield a 1 M ammonium sulfate concentration. The pH is adjusted to 7 and is mixed until dissolved at room temperature. The conjugate reaction mixture is applied to a HIC column packed with phenyl resin. The unconjugated polysaccharide is eluted with 2 to 7 column volumes of 1 M ammonium sulfate solution. The conjugate is eluted with WFI. In this and subsequent examples, the HIC purification of the conjugate can provide a product in which less than 20% of the polysaccharide by mass is free (unconjugated) polysaccharide. The conjugate eluate is diafiltered against 10 volume exchanges of 50mM sodium acetate, pH 6.0, using a 100 kDa MWCO PES membrane. The final filtration of the purified conjugate is performed using a 0.2 micron membrane and the conjugate is stored at 1 - 5°C.

### 2. Preparation of Group C Conjugates

### Example 2A

Group C purified capsular polysaccharide is dissolved in physiological saline to a target concentration of 10 mg/mL. The solution is mixed until dissolved. The temperature of the polysaccharide solution is adjusted to 37°C and sodium hydroxide (NaOH) is added to a target final concentration of 100 mM NaOH. The solution is mixed and incubated for 20 minutes, providing partial de-O-acetylation such that the polysaccharide in the final conjugate will have an O-acetylation level of 0.8 to 1.4 µmol OAc/mg polysaccharide and/or a reduction of 50% to 60% relative to the O-acetylation level of the starting material. Native MenC polysaccharide has two potential O-acetylation positions per monosaccharide repeat unit, and generally has an overall O-acetylation level of 40-45% for all possible O-acetylation sites. A 50% reduction in O-acetyl groups relative to the starting material will give an overall O-acetylation level (of all possible O-acetylation sites) of less than 25%.

The pH is adjusted to 6 and the temperature is decreased to 15°C. The dissolved polysaccharide is activated by the addition of sodium meta periodate (FIG. 2B) such that the target concentration is 2 mM. The pH is adjusted to 6 and the solution is mixed at 15°C. The periodate oxidizes and cleaves at adjacent diol positions, giving aldehyde-terminated chains. The reaction is mixed until the mean molecular size is reduced to between 50,000 and 100,000 Dalton, as determined by HPSEC. The reducing activity (reflecting the amount of aldehydes) is 40 to 100 nmol/mg polysaccharide. The reaction is quenched by adding glycerol in an amount of 0.5 mL glycerol per gram of polysaccharide and mixing for a minimum of 5 minutes. The polysaccharide is initially concentrated by ultrafiltration using a 5 kDa MWCO regenerated cellulose filter and then diafiltered against 8 - 12 volume exchanges of 50 mM sodium acetate buffer, pH 6.0. The material is further concentrated to a target concentration of 50mg/mL. The depolymerized/activated polysaccharide is filtered and stored.

Purified Tetanus Toxoid protein is concentrated on a 10 kDa MWCO PES membrane to a target final concentration of up to 100 mg/mL and then passed through a 0.2 micron filter. The filtered protein solution is stored at 1-5°C. The depolymerized/activated polysaccharide and concentrated Tetanus protein are mixed together, in a mass ratio of 0.5:1, 1:1, 2:1, 3:1, 4:1, or 5:1 (polysaccharide:protein). An aliquot of 100 mg/mL of sodium cyanoborohydride in 2.0 M phosphate buffer is added to the polysaccharide-protein mixture such that the sodium cyanoborohydride is 10 mg/mL and the phosphate buffer is 200 mM, pH 8.0. Saline is added to adjust concentrations, e.g., to a target of 15-50mg/mL for polysaccharide. The reaction (FIG. 2C) is mixed at 37°C for 16 - 30 hours. The reaction is diluted 1:2 with 6 mM phosphate buffered saline (PBS). An aliquot of 100 mg/mL sodium borohydride in 6 mM PBS is added to the reaction mixture to obtain a target 0.5 mg of sodium borohydride per mL of reaction volume. The reaction is mixed for a minimum of 15 minutes at room temperature. The sodium borohydride caps unreacted aldehydes by reducing them to alcohols, giving a terminal unlinked saccharide with a primary hydroxyl at the 7 position, or wherein the reducing end is modified with a (2-hydroxy)ethoxy. Products (terminal saccharides not shown) are illustrated in FIG. 2D and FIG. 2A. The conjugation solution is diafiltered against 10 volume exchanges of 6 mM PBS on a 50 kDa MWCO PES membrane. The solution is stored at 1-5°C.

Ammonium sulfate is added to the conjugate reaction to yield a 1 M ammonium sulfate concentration. The pH is adjusted to 7 and is mixed until dissolved at room temperature. The conjugate reaction mixture is applied to a HIC column packed with phenyl resin. The unconjugated polysaccharide is eluted with 2 to 7 column volumes of 1 M ammonium sulfate solution. The conjugate is eluted with WFI. In this and subsequent examples, the HIC purification of the conjugate can provide a product in which less than 20% of the polysaccharide by mass is free (unconjugated) polysaccharide. The conjugate eluate is diafiltered against 10 volume exchanges of 50mM sodium acetate, pH 6.0, using a 100 kDa MWCO PES membrane. The final filtration of the purified conjugate is performed using a 0.2 micron membrane and the conjugate is stored at 1 - 5°C.

### Example 2B

Group C purified capsular polysaccharide is dissolved in physiological saline to a target concentration of 10 mg/mL. The solution is mixed until dissolved. The pH is adjusted to 6.0 and the temperature is changed to 15°C. The dissolved polysaccharide is activated by the addition of sodium meta periodate (FIG. 2B) such that the target concentration is 2 mM. The reaction is mixed until the mean molecular size is between 50,000 and 100,000 Dalton, as determined by HPSEC. The reaction is quenched by adding glycerol in an amount of 0.5 mL glycerol per gram of polysaccharide and mixing for a minimum of 5 minutes. The polysaccharide is initially concentrated by ultrafiltration using a 5 kDa MWCO regenerated cellulose filter and then diafiltered against 8 - 12 volume exchanges of 50 mM sodium acetate buffer, pH 6.0. The material is further concentrated to a target concentration of 50mg/mL. The depolymerized/activated polysaccharide is filtered and is stored at 1-5°C.

Purified Tetanus Toxoid protein is concentrated on a 10 kDa MWCO PES membrane to a target final concentration of up to 100 mg/mL and then passed through a 0.2 micron filter. The filtered protein solution is stored at 1-5°C. The depolymerized/activated polysaccharide and concentrated Tetanus protein are mixed together, in a mole ratio of 0.5:1, 1:1, 2:1, 3:1, 4:1, or 5:1 (polysaccharide:protein). An aliquot of 100 mg/mL of sodium cyanoborohydride in 2.0 M phosphate buffer is added to the polysaccharide-protein mixture such that the sodium cyanoborohydride is 10 mg/mL and the phosphate buffer is 200 mM, pH 8.0. Saline is added to adjust concentrations, e.g., to a target of 15-50mg/mL for polysaccharide. The reaction (FIG. 2C) is mixed at 37°C for 16 - 30 hours. The reaction is diluted 1:2 with 6 mM phosphate buffered saline (PBS). An aliquot of 100 mg/mL sodium borohydride in 6 mM PBS is added to the reaction mixture to obtain a target 0.5 mg of sodium borohydride per mL of reaction volume. The reaction is mixed for a minimum of 15 minutes at room temperature. The sodium borohydride caps unreacted aldehydes by reducing them to alcohols, giving a terminal unlinked saccharide with a primary hydroxyl at the 7 position, or wherein the reducing end is modified with a (2-hydroxy)ethoxy. Products (terminal saccharides not shown) are illustrated in FIG. 2D and FIG. 2A. The conjugation solution is diafiltered against 10 volume exchanges of 6 mM PBS on a 50 kDa MWCO PES membrane. The solution is stored at 1-5°C.

Ammonium sulfate is added to the conjugate reaction to yield a 1 M ammonium sulfate concentration. The pH is adjusted to 7 and is mixed until dissolved at room temperature. The conjugate reaction mixture is applied to a HIC column packed with phenyl resin. The unconjugated polysaccharide is eluted with 2 to 7 column volumes of 1 M ammonium sulfate solution. The conjugate is eluted with WFI. In this and subsequent examples, the HIC purification of the conjugate can provide a product in which less than 20% of the polysaccharide by mass is free (unconjugated) polysaccharide. The conjugate eluate is diafiltered against 10 volume exchanges of 50mM sodium acetate, pH 6.0, using a 100 kDa MWCO PES membrane. The final filtration of the purified conjugate is performed using a 0.2 micron membrane and the conjugate is stored at 1 - 5°C.

### 3. Preparation of Group W-135 and Y Conjugates

Group W-135 purified capsular polysaccharide is dissolved in sodium acetate buffer to a target concentration of 10 mg/mL. The solution is mixed until dissolved. The polysaccharide solution is heated to 50 - 70°C using a jacketed heat exchanger. The pH is adjusted to 4.5. The reaction (FIG. 4A, step 1) is allowed to mix until the mean molecular size is 150,000 Dalton, as determined by HPSEC. The reaction mixture is cooled to 1-5°C. Sodium meta periodate is added to the polysaccharide solution such that the target meta periodate concentration is 2 mM (FIG. 4A, step 2). The pH is adjusted to 6.0 and the solution is mixed for 60 minutes between 0 and 5°C. The periodate oxidizes and cleaves at adjacent diol positions, giving aldehydes, e.g., at the 7-position of a sialic acid residue as shown in FIG. 4A. The reducing activity (reflecting the amount of aldehydes) is 60 to 150 nmol/mg polysaccharide. The reaction is quenched by adding 0.5 mL of glycerol per gram of polysaccharide and mixing for a minimum of 5 minutes. The polysaccharide is concentrated by ultrafiltration using a 10kDa MWCO regenerated cellulose filter and then diafiltered against 10 volume exchanges of 50 mM sodium acetate buffer, pH 6.0. The material is further concentrated to a target concentration of 50 mg/mL. The depolymerized/activated polysaccharide is filtered and stored at 1-5°C.

Purified Tetanus Toxoid protein is concentrated on a 10 kDa MWCO PES membrane to a target final concentration of up to 100 mg/mL and then passed through a 0.2 micron filter and is stored at 1-5°C. The depolymerized/activated polysaccharide and concentrated Tetanus protein are mixed together in a mass ratio of 0.5:1, 1:1, 2:1, 3:1, 4:1, or 5:1 (polysaccharide:protein). An aliquot of 100 mg/mL of sodium cyanoborohydride in 2.0 M phosphate buffer is added to the polysaccharide-protein mixture such that the sodium cyanoborohydride is 10 mg/mL and the phosphate buffer is 200 mM, pH 9.0. Saline is added to adjust target concentration, e.g., to a target of 15-50mg/mL for polysaccharide. The reaction (FIG. 4B) is mixed at room temperature overnight.

The reaction is diluted 1:2 with 6 mM phosphate buffered saline (PBS). An aliquot of 100 mg/mL sodium borohydride in 6 mM PBS is added to the reaction mixture to obtain a target 0.5 mg of sodium borohydride per mL of reaction volume. The reaction is mixed for a minimum of 15 minutes at room temperature. The sodium borohydride caps unreacted aldehydes by reducing them to alcohols. Products are shown in FIG. 4C and FIG. 3.

Ammonium sulfate is added to the conjugate reaction to yield a 1 M ammonium sulfate concentration. The pH is adjusted to 7 and is mixed until dissolved at room temperature. The conjugate reaction mixture is applied to a HIC column packed with phenyl resin. The unconjugated polysaccharide is eluted with 2 to 7 column volumes of 1 M ammonium sulfate solution. The conjugate is eluted with WFI. The conjugate eluate is diafiltered against 10 volume exchanges of 50mM sodium acetate, pH 6.0, using a 100 kDa MWCO PES membrane. The final filtration of the purified conjugate is performed using a 0.2 micron membrane and the conjugate is stored at 1 - 5°C. The same process can be used for Group Y purified capsular polysaccharide

### 4. Formulation of Quadrivalent Vaccines

### Example 4A

A quadrivalent MenACYW-TT conjugate vaccine is formulated from the 4 monovalent PS-protein conjugates prepared as described in Examples 1A, 2A, and 3-4 and diluted in a sodium acetate buffered saline solution to final concentration of 10 µg PS/serogroup/0.5 mL. That is, a 0.5 mL dose of MenACYW conjugate vaccine contains 10 µg of each of the meningococcal PS serogroups A, C, Y, and W-135, conjugated to 45 to 80 µg total of tetanus toxoid protein (the actual quantity of tetanus toxoid protein is dependent on the particular PS-to-protein ratios of the monovalent bulk concentrate lots used in the formulations).

Each 0.5 mL dose of MenACYW conjugate vaccine is formulated in a 30 mM sodium acetate-buffered pH 6.0 saline solution.

### Example 4B

A quadrivalent MenACYW-TT conjugate vaccine is formulated from the 4 monovalent PS-protein conjugates prepared as described in Examples 1A, 2B, and 3-4 and diluted in a sodium acetate buffered saline solution to final concentration of 10 µg PS/serogroup/0.5 mL. That is, a 0.5 mL dose of MenACYW conjugate vaccine contains 10 µg of each of the meningococcal PS serogroups A, C, Y, and W-135, conjugated to 45 to 80 µg total of tetanus toxoid protein (the actual quantity of tetanus toxoid protein is dependent on the particular PS-to-protein ratios of the monovalent bulk concentrate lots used in the formulations).

Each 0.5 mL dose of MenACYW conjugate vaccine is formulated in a 30 mM sodium acetate-buffered pH 6.0 saline solution.

### Example 4C

A quadrivalent MenACYW-TT conjugate vaccine is formulated from the 4 monovalent PS-protein conjugates prepared as described in Examples 1B, 2B, and 3-4 and diluted in a sodium acetate buffered saline solution to final concentration of 10 µg PS/serogroup/0.5 mL. That is, a 0.5 mL dose of MenACYW conjugate vaccine contains 10 µg of each of the meningococcal PS serogroups A, C, Y, and W-135, conjugated to 45 to 80 µg total of tetanus toxoid protein (the actual quantity of tetanus toxoid protein is dependent on the particular PS-to-protein ratios of the monovalent bulk concentrate lots used in the formulations).

Each 0.5 mL dose of MenACYW conjugate vaccine is formulated in a 30 mM sodium acetate-buffered pH 6.0 saline solution.

### 5. Phase II Clinical Trial - Safety and immunogenicity of MenACYW-TT administered in combination with Gardasil™ to healthy meningococcal vaccine naive adolescents (10-18 years)

As described herein, a quadrivalent MenACYW-TT conjugate formulated according to Example 4A and an HPV vaccine, Gardasil™ (quadrivalent human papillomavirus [type 6, 11, 16, 18] (HPV) recombinant vaccine), were used in a clinical study to evaluate safety and immunogenicity when administered in combination.

This phase II study compared safety and immunogenicity of a single dose (10 µg polysaccharide per serogroup, conjugated to 65 µg TT total, in 0.67% NaCl/30 mM sodium acetate buffered at pH 6.0) of MenACYW-TT administered alone (group 1) or coadministered together with Tdap/Adacel® and HPV/Gardasil® (group 3). As noted above, Gardasil® contains L1 proteins from HPV Types 6, 11, 16, and 18. Menveo® (Meningococcal (Groups A, C, Y and W-135) Oligosaccharide Diphtheria CRM197 Conjugate Vaccine, referred to herein as "MenACYW-CRM₁₉₇", a licensed quadrivalent meningococcal conjugate vaccine, was administered to a control group (group 2). The results were also compared to administration of Tdap/Adacel® and HPV/Gardasil® alone (group 4). The route of administration was intramuscular and subjects were adolescents aged 10-17 years. MenACYW-CRM₁₉₇, Tdap/Adacel®, and HPV/Gardasil® vaccines were administered according to label instructions, i.e., via intramuscular injection. MenACYW-TT (for groups 1 and 3) or MenACYW-CRM₁₉₇ (for group 2) was generally administered in the deltoid muscle of the right arm. Tdap/Adacel® and HPV/Gardasil® (for groups 3 and 4) were generally administered in the deltoid muscle of the left arm. The four study groups are summarized and characterized in Table 1. Subjects received a single dose of MenACYW-TT, MenACYW-CRM₁₉₇, and/or Tdap/Adacel®, depending on group assignments, on day 0 (D0). Three doses of HPV/Gardasil® were given as indicated below.

| **Table 1: Study groups** | | | | | |
|---|---|---|---|---|---|
| Group | n | Treatment | Males | Females | Mean, Median age (yrs) |
| 1 | 503 | MenACYW-TT | 243 | 260 | 11.4, 11.1 |
| 2 | 501 | MenACYW-CRM₁₉₇ | 272 | 229 | 11.4, 11.2 |
| 3 | 392 | MenACYW-TT with Tdap/Adacel® and HPV*/Gardasil® | 201 | 191 | 11.3, 11.1 |
| 4 | 296 | Tdap/Adacel® and HPV*/Gardasil® | 155 | 141 | 11.4, 11.1 |

| | | | | | |
|---|---|---|---|---|---|
| *: First dose of HPV vaccine was given on DO; HPV Dose 2 and Dose 3 were given 2 and 6 months, respectively, after Dose 1. | | | | | |

A total of 74 subjects (4.3%) did not complete the trial: 10 (2.0%) in Group 1, 7 (1.4%) in Group 2, 27 (6.7%) in Group 3, and 30 (10.0%) in Group 4. The most frequently reported reasons for discontinuation were: voluntary withdrawal not due to an adverse event, lost to follow-up, and non-compliance with the protocol. There were no early terminations due to an SAE or other AE.

Serum bactericidal assays with human complement (hSBA) were used to measure antibodies against meningococcal serogroups A, C, W and Y at baseline and 30 days after the dose. The LLOQ of the bactericidal assays was 1:4. hSBA data were collected for 463 members of group 1, 464 members of group 2, and 360 members of group 3. hSBA results are in Table 2, in which % subjects indicate the percentage of subjects with a positive seroresponse, i.e., post vaccination hSBA ≥ 1:8 for subjects with pre-vaccination hSBA titers <1:8, or at least a 4-fold increase in hSBA titers from pre to post-vaccination for subjects with pre-vaccination titers ≥ 1:8. A greater percentage of subjects showed a positive seroresponse with MenACYW-TT than with MenACYW-CRM₁₉₇ for all four serogroups.

| **Table 2. MenACWY hSBA Results** | | | | | | |
|---|---|---|---|---|---|---|
| | Group 1 (MenACYW-TT) | | Group 2 (MenACYW-CRM₁₉₇) | | Group 3 (MenACYW-TT + Tdap + HPV | |
| | (N=463) | | (N=464) | | (N=360) | |
| Serogroup | % subjects | 95%CI | % subjects | 95%CI | % subjects | 95%CI |
| A | 75.6 | 71.4; 79.4 | 66.4 | 61.9; 70.7 | 80.6 | 76.1; 84.5 |
| C | 97.2 | 95.2; 98.5 | 72.6 | 68.3; 76.6 | 97.2 | 95.0; 98.7 |
| Y | 97.0 | 95.0; 98.3 | 80.8 | 76.9; 84.3 | 95.6 | 92.9; 97.4 |
| W | 86.2 | 82.7; 89.2 | 66.6 | 62.1; 70.9 | 83.9 | 79.7; 87.5 |

The difference in seroresponse frequency between groups 1 and 2 is shown in Table 3 along with the 95% confidence interval thereof.

| **Table 3. Group 1 - Group 2 differential seroresponse** | | |
|---|---|---|
| Serogroup | Difference (% subjects) | 95% CI |
| A | 9.2 | 3.4; 15.0 |
| C | 24.6 | 20.3; 29.0 |
| Y | 16.2 | 12.3; 20.2 |
| W | 19.6 | 14.2; 24.8 |

The difference in seroresponse frequency between groups 1 and 3 was not significant at 95% confidence, consistent with the conclusion that MenACYW-TT efficacy is not affected by coadministration with Tdap/Adacel® and HPV/Gardasil®.

Table 4 shows hSBA results expressed as geometric mean titers (GMT) at day 0 (D0) and day 30 (D30), along with 95% confidence intervals.

| **Table 4. hSBA Geometric Mean Titers** | | | | | | | |
|---|---|---|---|---|---|---|---|
| | | Group 1 | | Group 2 | | Group 3 | |
| | | (N=463) | | (N=464) | | (N=360) | |
| Serogroup | | GMT | 95%CI | GMT | 95%CI | GMT | 95%CI |
| A | D0 | 6.19 | 5.62; 6.83 | 5.75 | 5.24; 6.31 | 5.34 | 4.8; 5.94 |
| | D30 | 44.1 | 39.2; 49.6 | 35.2 | 30.3; 41.0 | 47.9 | 41.7; 55.0 |
| C | D0 | 3.36 | 3.12; 3.62 | 3.08 | 2.88; 3.30 | 3.38 | 3.13; 3.64 |
| | D30 | 387 | 329; 456 | 51.4 | 41.2; 64.2 | 335 | 280; 399 |
| Y | D0 | 2.33 | 2.23; 2.43 | 2.41 | 2.28; 2.54 | 2.46 | 2.32; 2.62 |
| | D30 | 75.7 | 66.2; 86.5 | 27.6 | 23.8; 32.1 | 77.3 | 66.5; 89.9 |
| W | D0 | 5.17 | 4.67; 5.73 | 5.35 | 4.82; 5.94 | 5.87 | 5.22; 6.60 |
| | D30 | 86.9 | 77.8; 97 | 36.0 | 31.5; 41.0 | 91 | 80.2; 103 |

Thus, coadministration of HPV/Gardasil® is considered not to interfere with the immunogenicity of MenACYW-TT.

Immune responses to HPV were compared for groups 3 (coadministration with MenACYW-TT) and 4 (no MenACYW-TT). Anti-HPV 6, 11, 16, and 18 immunoglobulin levels were measured using a competitive Luminex immunoassay (cLIA). Serum samples were evaluated for their ability to prevent virus-like particle (VLP) binding by a type-specific neutralizing monoclonal antibody (mAb). The strength of the antibody response is inversely proportional to the detection of the mAb binding signal. Samples were obtained 30 days after the third dose of HPV vaccine.

Table 5 shows HPV serogroup-specific seroconversion in Groups 3 and 4. Table 6 shows HPV serogroup-specific geometric mean titers (GMTs) in Groups 3 and 4.

| **Table 5. HPV Seroconversion** | | | | |
|---|---|---|---|---|
| | Group 3 | | Group 4 | |
| | (N=360) | | (n = 263) | |
| HPV Type | Seroconversion (%) | 95%CI | Seroconversion (%) | 95%CI |
| 6 | 97.5 | 94.7; 99.1 | 95.7 | 91.4; 98.3 |
| 11 | 99.6 | 97.7; 100 | 98.8 | 95.7; 99.9 |
| 16 | 99.2 | 97.0; 99.9 | 98.8 | 95.7; 99.9 |
| 18 | 99.2 | 97.0; 99.9 | 98.8 | 95.7; 99.9 |

| **Table 6. HPV GMTs** | | | | |
|---|---|---|---|---|
| | Group 3 | | Group 4 | |
| | (N=360) | | (n = 263) | |
| HPV Type | GMT | 95%CI | GMT | 95%CI |
| 6 | 800 | 664; 963 | 800 | 656; 975 |
| 11 | 1492 | 1291; 1924 | 1402 | 1211; 1623 |
| 16 | 6002 | 5025; 7170 | 6395 | 5402; 7571 |
| 18 | 1271 | 1077; 1499 | 1118 | 933; 1340 |

Thus, coadministration of MenACYW-TT is considered not to interfere with the immunogenicity of HPV/Gardasil®.

Immune responses to diphtheria and tetanus were compared for all groups. Results are shown in Table 7, expressed as geometric mean concentration (GMC); % subjects with ≥ 0.1 IU/mL; and % subjects with ≥ 1.0 IU/mL of the anti-tetanus and anti-diphtheria antibody concentrations.

| **Table 7. Post-vaccination geometric means and titers for Diphtheria and Tetanus** | | | | | | |
|---|---|---|---|---|---|---|
| | Diphtheria | | | Tetanus | | |
| | GMC | ≥ 0.1 IU/mL (%) | ≥ 1.0 IU/mL (%) | GMC | ≥ 0.1 IU/mL (%) | ≥ 1.0 IU/mL (%) |
| Group 1 (N=463) | 0.152 | 57.4 | 7.4 | 21.4 | 100 | 97.9 |
| Group 2 (N=464) | 35.4 | 100 | 98.9 | 0.346 | 90.1 | 18.7 |
| Group 3 (N=360) | 11.9 | 99.4 | 97.8 | 29.0 | 99.7 | 99.7 |
| Group 4 (N=263) | 15.7 | 99.6 | 98.9 | 14.7 | 100 | 99.6 |

Results were consistent with the conclusion that coadministration of MenACYW-TT with Tdap/Adacel® as in group 3 did not interfere with the immunogenicity of the latter (cf. group 4 results).

Vaccine responses were also characterized with respect to the following antigens: pertussis toxin (PT), pertussis filamentous hemagglutinin (FHA), pertussis pertactin (PRN) and pertussis fimbrial antigen (FIM). See Table 8.

| **Table 8. Responses to PT, FHA, PRN and FIM antigens.** | | | | | | |
|---|---|---|---|---|---|---|
| | Group 3 | | | Group 4 | | |
| | (N=360) | | | (n = 263) | | |
| Ag | GMT/GMC | 95% CI | Vaccine Response (%) | GMT/GMC | 95% CI | Vaccine Response (%) |
| PT | 37.5 | 33.8; 41.7 | 67.3 | 44.4 | 39.5; 49.9 | 78.2 |
| FHA | 180 | 168; 194 | 92.1 | 242 | 218; 268 | 89.4 |
| PRN | 200 | 177; 225 | 94.7 | 265 | 231; 304 | 96.6 |
| FIM | 339 | 285; 403 | 92.2 | 499 | 414; 601 | 95.4 |

The following were observed with respect to safety: occurrence, nature, duration, intensity, and relationship to vaccination of any unsolicited systemic adverse events (AEs) reported in the 30 minutes after vaccination; occurrence, time to onset, number of days of occurrence, intensity, action taken, and whether the reaction led to early termination from the study, of solicited injection site reactions occurring up to 7 days after DO vaccination(s); occurrence, time to onset, number of days of occurrence, intensity, action taken, and whether the reaction led to early termination from the study, of solicited systemic reactions occurring up to 7 days after DO vaccination(s); occurrence, nature, time to onset, duration, intensity, action taken, relationship to vaccination (for systemic AEs only), and whether the event led to early termination from the study, of unsolicited AEs up to 23-37 days after DO vaccination(s); and occurrence, nature, time to onset, duration, seriousness criteria, relationship to vaccination, outcome, and whether the serious adverse event (SAE) led to early termination from the study, of SAEs throughout the trial up to 180 days (Group 1 and Group 2) or 210 days (Group 3 and Group 4) after DO vaccination(s). Solicited systemic reactions included fever, myalgia, and headache. Solicited injection site reactions included pain, erythema, and swelling.

The percentages of subjects reporting at least 1 solicited reaction between DO and D07 were comparable between MenACYW-TT conjugate vaccine and MENVEO®: 63.5% (315/496) of subjects in Group 1 and 64.2% (316/492) in Group 2, respectively. The percentages of subjects reporting at least 1 solicited reaction were comparable between subjects who received MenACYW-TT conjugate vaccine concomitantly with Tdap and HPV versus Tdap and HPV alone: 88.9% (345/388) in Group 3 and 89.0% (258/290) in Group 4, respectively. The percentages of subjects who reported at least 1 solicited injection site reaction were comparable between Group 1, Group 2, and Group 3: 46.6% (231/496), 45.7% (225/492), and 49.0% (190/388), respectively. No increase in local reactogenicity for the MenACYW-TT conjugate vaccine was seen when MenACYW-TT conjugate vaccine was given concomitantly with Tdap and HPV (Group 3) versus when MenACYW-TT conjugate vaccine was given alone (Group 1).

The most frequently reported solicited injection site reaction was pain, reported by 45.2% (224/496) of subjects in Group 1, 42.5% (209/492) of subjects in Group 2, and 47.2% (183/388) of subjects in Group 3, followed by injection site erythema which was reported by 5.0% (25/496) of subjects in Group 1, 7.5% (37/491) of subjects in Group 2, and 3.9% (15/388) of subjects in Group 3, and injection site swelling which was reported by 5.4% (27/496) of subjects in Group 1, 6.5% (32/491) of subjects in Group 2, and 4.4% (17/388) of subjects in Group 3. The majority of reactions at the MenACYW-TT conjugate vaccine or MENVEO® injection sites were of Grade 1 or 2 intensity, started between DO and D03, and lasted 1 to 3 days. The percentages of subjects with any Grade 3 injection site reaction at the MenACYW-TT conjugate vaccine or MENVEO® injection site were 1.8% (9/496) in Group 1, 2.2% (11/492) in Group 2, and 2.8% (11/388) in Group 3. The percentages of subjects with Grade 3 pain at the MenACYW-TT conjugate vaccine or MENVEO® injection site were 1.4% (7/496) in Group 1, 1.0% (5/492) in Group 2, and 2.3% (9/388) in Group 3. The percentages of subjects with Grade 3 erythema were 0.4% (2/496) in Group 1, 1.2% (6/491) in Group 2, and 0.5% (2/388) in Group 3. The percentages of subjects with Grade 3 swelling were 0.2% (1/496) in Group 1, 0.4% (2/491) in Group 2, and 0.3% (1/388) in Group 3. Intensity grades generally have the following meanings. Grade 1: No interference with activity. Grade 2: Some interference with activity. Grade 3: Significant; prevents daily activity.

At the HPV vaccination site, the percentages of subjects who reported at least 1 solicited injection site reaction were 74.2% (288/388) in Group 3 versus 71.3% (206/289) in Group 4. The most frequently reported solicited injection site reaction was pain, reported by 74.2% (288/388) of subjects in Group 3 and 69.6% (201/289) of subjects in Group 4. Injection site swelling was reported by 6.7% (26/388) of subjects in Group 3 and 8.0% (23/289) of subjects in Group 4. Injection site erythema was reported by 8.0% (31/388) of subjects in Group 3 and 5.5% (16/289) of subjects in Group 4.

The percentages of subjects reporting at least 1 solicited systemic reaction after vaccination were comparable between Group 1 (52.0% [258/496]) and Group 2 (51.0% [251/492]). Myalgia was the most commonly reported solicited systemic reaction followed by headache and malaise with very few reports of fever. Myalgia was reported in 35.3% (175/496) of subjects in Group 1 and 35.2% (173/492) of subjects in Group 2. Headache was reported in 30.2% (150/496) of subjects in Group 1 and 30.9% (152/492) of subjects in Group 2. Malaise was reported in 26.0% (129/496) of subjects in Group 1 and 26.4% (130/492) of subjects in Group 2. Fever was reported in 1.4% (7/494) of subjects in Group 1 and 1.2% (6/488) of subjects in Group 2.

The percentages of subjects with at least 1 solicited systemic reaction after vaccination were comparable between Group 3 (70.6% [274/388]) and Group 4 (65.9% [191/290]). Myalgia was the most commonly reported solicited systemic reaction: 61.3% (238/388) of subjects in Group 3 and 55.4% (160/289) of subjects in Group 4. Headache was reported in 33.8% (131/388) of subjects in Group 3, and 29.0% (84/290) of subjects in Group 4. Malaise was reported in 29.1% (113/388) of subjects in Group 3, and 27.9% (81/290) of subjects in Group 4. Fever was reported in 1.6% (6/387) of subjects in Group 3, and 0.7% (2/286) of subjects in Group 4. Overall, most solicited systemic reactions were of Grade 1 or Grade 2 intensity, started between DO and D03, and lasted 1 to 3 days.

Overall, the percentages of subjects who reported Grade 3 solicited systemic reactions were comparable between Group 1 (3.8% [19/496]) and Group 2 (4.3% [21/492]). The percentages of subjects who reported Grade 3 solicited systemic reactions were comparable between Group 3 (7.5% [29/388]) and Group 4 (5.5% [16/290]). The most frequently reported Grade 3 solicited systemic reaction was myalgia followed by malaise and headache. The percentages of subjects who reported Grade 3 myalgia were comparable between Group 1 (1.6% [8/496]) and Group 2 (1.8% [9/492]) and between Group 3 (4.6% [18/388]) and Group 4 (3.8% [11/289]). The percentages of subjects who reported Grade 3 malaise were comparable between Group 1 (2.2% [11/496]) and Group 2 (2.8% [14/492]). Malaise was reported more frequently in Group 3 (2.6% [10/388]) than in Group 4 (1.7% [5/290]). The percentages of subjects who reported Grade 3 headache were the same for both Group 1 (1.8% [9/496]) and Group 2 (1.8% [9/492]). Headache was reported more frequently in Group 3 (2.8% [11/388]) than in Group 4 (1.7% [5/290]).

Overall, the percentages of subjects reporting at least 1 unsolicited AE between DO and D30 were comparable across the 4 study groups: 22.9% (115/503) of subjects in Group 1 and 25.7% (129/501) in Group 2; 26.0% (102/392) in Group 3 and 22.6% (67/296) in Group 4. Few subjects reported immediate unsolicited AEs: 0.6% (3/503) of subjects in Group 1, 0.2% (1/501) of subjects in Group 2, 0.8% (3/392) of subjects in Group 3, and 0.7% (2/296) of subjects in Group 4. There were no immediate SAEs, including any anaphylactic or life-threatening events. Twelve immediate unsolicited AEs were reported in 9 subjects at 23-37 days. One subject reported 1 immediate unsolicited AE at six months after DO vaccination(s).

The percentages of subjects who reported at least 1 unsolicited non-serious injection site AR after DO vaccination(s) were comparable between Group 1 and Group 2: 1.4% (7/503) and 1.6% (8/501), respectively; there was a numerically higher percentage of subjects who reported at least 1 unsolicited non-serious injection site AR in Group 3 than in Group 4: 4.3% (17/392) and 2.0% (6/296), respectively. The most commonly reported unsolicited injection site reaction was pruritus, reported in 14 subjects, followed by bruising, reported in 13 subjects. These unsolicited injection site reactions may occur after any vaccination in general.

The percentages of subjects who reported at least 1 unsolicited non-serious injection site AR at the MenACYW-TT conjugate vaccine or MENVEO® injection sites were comparable: 1.4% (7/503) in Group 1, 1.6% (8/501) in Group 2, and 1.8% (7/392) in Group 3. One subject in Group 2 reported 1 Grade 3 unsolicited non-serious injection site AR of injection site warmth which started on D01, lasted 4 days, and resolved spontaneously. No action was taken. No Grade 3 unsolicited non-serious injection site ARs were reported in Group 1 or at the MenACYW-TT conjugate vaccine injection site in Group 3.

The percentages of subjects reporting at least 1 unsolicited non-serious AE within 30 days were comparable across the 4 study groups: 22.7% (114/503) of subjects in Group 1, 25.5% (128/501) of subjects in Group 2, 26.0% (102/392) of subjects in Group 3, and 22.3% (66/296) of subjects in Group 4. Most frequently reported were infections and infestations (7.2% [36/503] of subjects in Group 1, 8.0% [40/501] of subjects in Group 2, 8.2% [32/392] of subjects in Group 3, 6.1% [18/296] of subjects in Group 4); the most common type was upper respiratory tract infection.

Sixteen subjects reported SAEs during the trial period; 4 subjects reported SAEs within 30 days of vaccination on DO. None were considered as related to the vaccine, and none led to discontinuation from the study. All subjects recovered. No deaths were reported during the study period.

Vaccination with MenACYW-TT conjugate vaccine among adolescents was found to be safe, with no safety concerns identified when given alone or concomitantly with Tdap and HPV vaccines. The safety profile of MenACYW-TT conjugate vaccine administered alone was comparable to that of the licensed MENVEO® vaccine administered alone.

Although the foregoing invention has been described in some detail by way of illustration and example for purposes of clarity of understanding, the descriptions and examples should not be construed as limiting the scope of the invention.

## Claims

1. A *Neisseria meningitidis* vaccine composition for use in a method of immunization against *Neisseria meningitidis* serogroups A, C, Y, and W-135 and human papilloma virus, wherein:
the *Neisseria meningitidis* vaccine composition comprises:
a) a first conjugate of MenA capsular polysaccharide to tetanus toxoid;
b) a second conjugate of MenC capsular polysaccharide to tetanus toxoid;
c) a third conjugate of MenW-135 capsular polysaccharide to tetanus toxoid; and
d) a fourth conjugate of MenY capsular polysaccharide to tetanus toxoid;
the method comprises coadministering the *Neisseria meningitidis* vaccine composition and an HPV Type 18 L1 protein to a subject in need thereof; and
the administration of the *Neisseria meningitidis* vaccine composition does not interfere with the development of immunity against HPV Type 18.

2. The *Neisseria meningitidis* vaccine composition for use according to claim 1, wherein:
(i) the method further comprises coadministering an HPV Type 16 L1 protein with the *Neisseria meningitidis* vaccine composition; and/or
(ii) the method further comprises coadministering an HPV Type 6 L1 protein and/or an HPV Type 11 L1 protein with the *Neisseria meningitidis* vaccine composition.

3. The *Neisseria meningitidis* vaccine composition for use according to any one of the preceding claims, wherein:
(i) the administration of the *Neisseria meningitidis* vaccine composition does not interfere with the development of immunity against HPV Type 16;
(ii) the administration of the *Neisseria meningitidis* vaccine composition does not interfere with the development of immunity against HPV Type 6 and/or HPV Type 11; and/or
(iii) the administration of the HPV protein or proteins does not interfere with the development of immunity against *Neisseria meningitidis* serogroups A, C, Y, and/or W-135.

4. The *Neisseria meningitidis* vaccine composition for use according to any one of the preceding claims, wherein interference or non-interference with the development of immunity against HPV Type 18, 16, 11, and/or 6 and/or *Neisseria meningitidis* serogroups A, C, Y, and/or W-135 is determined by comparing geometric mean titers.

5. The *Neisseria meningitidis* vaccine composition for use according to any one of the preceding claims, wherein the coadministration of the *Neisseria meningitidis* vaccine composition and the HPV protein or proteins does not result in increased risk of injection site swelling relative to administration of the HPV protein or proteins without the *Neisseria meningitidis* vaccine composition.

6. The *Neisseria meningitidis* vaccine composition for use according to any one of the preceding claims, wherein the method further comprises coadministering a diphtheria-tetanus-pertussis vaccine with the *Neisseria meningitidis* vaccine composition, optionally wherein the diphtheria-tetanus-pertussis vaccine is Tetanus, diphtheria, acellular pertussis [Tdap] vaccine or DTaP5, optionally wherein the administration of the diphtheria-tetanus-pertussis vaccine does not interfere with the development of immunity against *Neisseria meningitidis* serogroups A, C, Y, and/or W-135.

7. The *Neisseria meningitidis* vaccine composition for use according to any one of the preceding claims, wherein:
(i) the second conjugate is a population comprising double-end-linked conjugated polysaccharides and single-end-linked conjugated polysaccharides which both are attached to the tetanus toxoid through a secondary amine, and/or the polysaccharides of the second conjugate have an O-acetylation level of 0.3 µmol/mg polysaccharide to 1.6 µmol/mg polysaccharide;
(ii) the second conjugate is a population comprising single-end-linked conjugated polysaccharides which are attached to the tetanus toxoid through a secondary amine, wherein the single-end-linked conjugated polysaccharides have a terminal unlinked saccharide, optionally wherein the terminal saccharide has a primary hydroxyl or secondary amine linkage at the 7 position, or wherein the reducing end is modified with a (2-hydroxy)ethoxy or secondary amine linkage;
(iii) the MenA capsular polysaccharide is attached to the tetanus toxoid through a linker comprising a carbamate, a spacer, and an amide, wherein the spacer is between the carbamate and the amide and comprises 2-10 linear carbons, and/or the first conjugate has a polysaccharide to tetanus toxoid mass ratio of 0.3 to 1.5;
(iv) the MenA capsular polysaccharide is attached to the tetanus toxoid through a linker comprising a carbamate, a spacer, and an amide, optionally wherein the spacer is between the carbamate and the amide and comprises 2-10 linear carbons;
(v) the MenC, MenW-135, and MenY capsular polysaccharides are attached to the tetanus toxoid through a secondary amine; and/or at least one of the conjugates has a weight average molecular weight ranging from 300 kDa to 1500 kDa;
(vi) one or more of the first, second, third, and fourth conjugates has a weight average molecular weight ranging from 300 kDa to 1500 kDa; and/or the composition comprises less than 20% free polysaccharide by weight relative to total polysaccharide; and/or
(vii) one or more of the first, second, third, and fourth conjugates have a polysaccharide to tetanus toxoid mass ratio of 0.3 to 1.5; and/or the composition comprises less than 20% free polysaccharide by weight relative to total polysaccharide;
optionally wherein molecular weight is determined by multi-angle light scattering (MALS).

8. The *Neisseria meningitidis* vaccine composition for use according to any one of the preceding claims, wherein the first, second, third, and/or fourth conjugates are a population comprising molecules with a molecular weight in the range of 700 kDa to 1400 kDa or 800 kDa to 1300 kDa. optionally wherein molecular weight is determined by multi-angle light scattering (MALS).

9. The *Neisseria meningitidis* vaccine composition for use according to any one of the preceding claims, wherein:
(i) the MenC polysaccharide has a degree of O-acetylation ranging from 0.6 to 1.5 µmol/mg polysaccharide or 0.8 to 1.4 µmol/mg polysaccharide;
(ii) the conjugate comprising MenC polysaccharide is a population comprising double-end-linked conjugated polysaccharides and single-end-linked conjugated polysaccharides, optionally wherein the single-end-linked polysaccharides of the second conjugate comprise a terminal unlinked saccharide, wherein the single-end-linked conjugated polysaccharides have a terminal unlinked saccharide, wherein the terminal saccharide has a primary hydroxyl at the 7 position, or wherein the reducing end is modified with a (2-hydroxy)ethoxy;
(iii) the conjugate comprising MenC polysaccharide comprises one or more modifications chosen from (a) a primary hydroxyl at the 7 position, (b) a (2-hydroxy)ethoxy at the reducing end, and (c) a conjugation to the tetanus toxoid, wherein the modifications are present at no less than 25 nmol/mg polysaccharide;
(iv) the conjugate of MenW-135 and/or MenY polysaccharide comprises one or more modifications chosen from (a) a primary hydroxyl at a position of a vicinal diol in a native MenW-135 or MenY polysaccharide and (b) a conjugation to the tetanus toxoid, wherein the modifications are present at no less than 60 nmol/mg polysaccharide;
(v) the MenC polysaccharide is reduced in size by 3x-8x relative to native MenC polysaccharide; and/or
(vi) the composition comprises less than 20% free polysaccharide by weight, less than 10% free polysaccharide by weight, less than 5% free polysaccharide by weight, or substantially lacks free polysaccharide.

10. The *Neisseria meningitidis* vaccine composition for use according to any one of the preceding claims, wherein:
(i) the conjugate of the MenA capsular polysaccharide to the tetanus toxoid has a polysaccharide to tetanus toxoid mass ratio of 0.5 to 1.5, 0.7 to 1.4, or 0.8 to 1.3;
(ii) the conjugate of the MenC capsular polysaccharide to the tetanus toxoid has a polysaccharide to tetanus toxoid mass ratio of 0.3 to 1.1 or 0.4 to 0.8;
(iii) the conjugate of the MenY capsular polysaccharide to the tetanus toxoid has a polysaccharide to tetanus toxoid mass ratio of 0.3 to 1.1, 0.5 to 1.3, or 0.5 to 0.9; and/or
(iv) the conjugate of MenW-135 capsular polysaccharide to the tetanus toxoid has a polysaccharide to tetanus toxoid mass ratio of 0.3 to 1.3 or 0.6 to 1.3.

11. The *Neisseria meningitidis* vaccine composition for use according to any one of the preceding claims, wherein the polysaccharide of the MenA, MenC, MenW-135, or MenY conjugate is attached to the tetanus toxoid through a linker, optionally wherein:
(i) the linker comprises 2-10 linear carbons;
(ii) the linker is present in the MenA, MenC, MenW-135, or MenY conjugate at a ratio of one linker per 10-100 saccharide repeat units or 20-60 saccharide repeat units; and/or
(iii) the linker comprises a spacer between a first carbonyl and a second carbonyl, and the spacer comprises 4-8 carbons.

12. The *Neisseria meningitidis* vaccine composition for use according to any one of the preceding claims, wherein:
(i) the MenA conjugate comprises a linker comprising a residue of a dihydrazide or a residue of adipic acid dihydrazide, optionally wherein the polysaccharide of the MenA conjugate is attached to the tetanus toxoid through a linker of formula I: 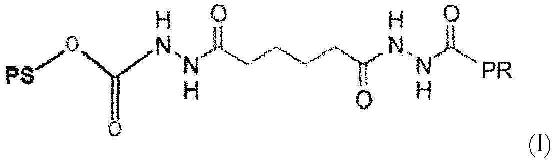 wherein PS indicates attachment to the polysaccharide and PR indicates attachment to the tetanus toxoid; and/or
(ii) the polysaccharide of the MenC, MenW-135, and/or MenY conjugate is attached to the tetanus toxoid as shown in formula II:
PR-NH-CH₂-PS (II)
wherein PS indicates attachment to the polysaccharide and PR indicates attachment to the tetanus toxoid.

13. The *Neisseria meningitidis* vaccine composition for use according to any one of the preceding claims, wherein the *Neisseria meningitidis* vaccine composition is a single unit dose composition comprising from 6 µg to 15 µg or 4 µg to 10 µg of each of the MenA, MenC, MenW-135, and MenY polysaccharides, and/or wherein the tetanus toxoid is present in the vaccine composition in an amount from 50 µg to 80 µg.

14. The *Neisseria meningitidis* vaccine composition for use according to any one of the preceding claims, wherein the HPV Type 18 L1 protein is administered as part of an HPV vaccine, optionally wherein the HPV vaccine comprises one, two, three, four, or all of:
(i) amorphous aluminum hydroxyphosphate sulfate adjuvant;
(ii) sodium chloride;
(iii) L-histidine;
(iv) polysorbate 80; and/or
(v) sodium borate;
and/or optionally wherein the HPV L1 protein or proteins are in the form of virus-like particles.

15. The *Neisseria meningitidis* vaccine composition for use according to claim 14, wherein the HPV vaccine comprises one, two, three, or all of:
(i) a 20 µg dose of HPV Type 6 L1 protein;
(ii) a 40 µg dose of HPV Type 11 L1 protein;
(iii) a 40 µg dose of HPV Type 16 L1 protein; and/or
(iv) a 20 µg dose of HPV Type 18 L1 protein;
optionally wherein the HPV vaccine is quadrivalent human papillomavirus [type 6, 11, 16, 18] (HPV) recombinant vaccine (Gardasil™).
